# EUROPEAN PATENT SPECIFICATION

(11) **EP 3 622 524 B1**
(45) Date of publication and mention of the grant of the patent: **06.08.2025**
(21) Application number: 19721182.4
(22) Date of filing: 12.04.2019
(51) Int. Cl.: G16B 40/20, G16B 20/20

(54) **VARIANT CLASSIFIER BASED ON DEEP NEURAL NETWORKS**
VARIANTENKLASSIERER AUF DER BASIS VON TIEFEN NEURONALEN NETZEN
CLASSIFICATEUR DE VARIANTES BASÉ SUR DES RÉSEAUX NEURONAUX PROFONDS

(30) Priority: 12.04.2018 US 201862656741 P; 02.05.2018 NL 2020861
(43) Date of publication of application: 18.03.2020
(60) Divisional application: 25185327.1
(73) Proprietor: Illumina, Inc., San Diego, CA 92122 (US)
(72) Inventor: WISE, Aaron, San Diego, California 92104 (US); KRUGLYAK, Kristina M., San Diego, California 92122 (US)
(74) Representative: Robinson, David Edward Ashdown
(86) International application number: PCT/US2019/027362
(87) International publication number: WO 2019/200338

(56) References cited:
- WO-A1-2018/006152
- CA-A1- 2 894 317
- REMI TORRACINTA ET AL: "Adaptive Somatic Mutations Calls with Deep Learning and Semi-Simulated Data", BIORXIV, 4 October 2016 (2016-10-04), XP055540696, Retrieved from the Internet <URL:https://www.biorxiv.org/content/biorxiv/early/2016/10/04/079087.full-text.pdf> DOI: 10.1101/079087
- CHRISTOF ANGERMUELLER ET AL: "Deep learning for computational biology", MOLECULAR SYSTEMS BIOLOGY, vol. 12, no. 7, 1 July 2016 (2016-07-01), GB, pages 878, XP055540697, ISSN: 1744-4292, DOI: 10.15252/msb.20156651

## Description

### PRIORITY APPLICATIONS

This application claims priority to or the benefit of the following applications:

US Provisional Patent Application No. 62/656,741, entitled "VARIANT CLASSIFIER BASED ON DEEP NEURAL NETWORKS," filed on April 12, 2018, (Atty. Docket No. ILLM 1007-1/IP-1681-PRV); and

Netherlands Application No. 2020861, entitled "VARIANT CLASSIFIER BASED ON DEEP NEURAL NETWORKS," filed on May 2, 2018, (Atty. Docket No. ILLM 1007-4/IP-1681-NL).

### FIELD OF THE TECHNOLOGY DISCLOSED

The technology disclosed relates to artificial intelligence type computers and digital data processing systems and corresponding data processing methods and products for emulation of intelligence (i.e., knowledge based systems, reasoning systems, and knowledge acquisition systems); and including systems for reasoning with uncertainty (e.g., fuzzy logic systems), adaptive systems, machine learning systems, and artificial neural networks. In particular, the technology disclosed relates to using deep neural networks such as convolutional neural networks (CNNs) and fully-connected neural networks (FCNNs) for analyzing data.

### BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

Next-generation sequencing has made large amounts of sequenced data available for variant classification. Sequenced data are highly correlated and have complex interdependencies, which has hindered the application of traditional classifiers like support vector machine to the variant classification task. Advanced classifiers that are capable of extracting high-level features from sequenced data are thus desired.

Deep neural networks are a type of artificial neural networks that use multiple nonlinear and complex transforming layers to successively model high-level features and provide feedback via backpropagation. Deep neural networks have evolved with the availability of large training datasets, the power of parallel and distributed computing, and sophisticated training algorithms. Deep neural networks have facilitated major advances in numerous domains such as computer vision, speech recognition, and natural language processing.

Convolutional neural networks and recurrent neural networks are components of deep neural networks. Convolutional neural networks have succeeded particularly in image recognition with an architecture that comprises convolution layers, nonlinear layers, and pooling layers. Recurrent neural networks are designed to utilize sequential information of input data with cyclic connections among building blocks like perceptrons, long short-term memory units, and gated recurrent units. In addition, many other emergent deep neural networks have been proposed for limited contexts, such as deep spatio-temporal neural networks, multi-dimensional recurrent neural networks, and convolutional auto-encoders.

The goal of training deep neural networks is optimization of the weight parameters in each layer, which gradually combines simpler features into complex features so that the most suitable hierarchical representations can be learned from data. A single cycle of the optimization process is organized as follows. First, given a training dataset, the forward pass sequentially computes the output in each layer and propagates the function signals forward through the network. In the final output layer, an objective loss function measures error between the inferenced outputs and the given labels. To minimize the training error, the backward pass uses the chain rule to backpropagate error signals and compute gradients with respect to all weights throughout the neural network. Finally, the weight parameters are updated using optimization algorithms based on stochastic gradient descent. Whereas batch gradient descent performs parameter updates for each complete dataset, stochastic gradient descent provides stochastic approximations by performing the updates for each small set of data examples. Several optimization algorithms stem from stochastic gradient descent. For example, the Adagrad and Adam training algorithms perform stochastic gradient descent while adaptively modifying learning rates based on update frequency and moments of the gradients for each parameter, respectively.

Another core element in the training of deep neural networks is regularization, which refers to strategies intended to avoid overfitting and thus achieve good generalization performance. For example, weight decay adds a penalty term to the objective loss function so that weight parameters converge to smaller absolute values. Dropout randomly removes hidden units from neural networks during training and can be considered an ensemble of possible subnetworks. To enhance the capabilities of dropout, a new activation function, maxout, and a variant of dropout for recurrent neural networks called rnnDrop have been proposed. Furthermore, batch normalization provides a new regularization method through normalization of scalar features for each activation within a mini-batch and learning each mean and variance as parameters.

Given that sequenced data are multi- and high-dimensional, deep neural networks have great promise for bioinformatics research because of their broad applicability and enhanced prediction power. Convolutional neural networks have been adapted to solve sequence-based problems in genomics such as motif discovery, pathogenic variant identification, and gene expression inference. A hallmark of convolutional neural networks is the use of convolution filters. Unlike traditional classification approaches that are based on elaborately-designed and manually-crafted features, convolution filters perform adaptive learning of features, analogous to a process of mapping raw input data to the informative representation of knowledge. In this sense, the convolution filters serve as a series of motif scanners, since a set of such filters is capable of recognizing relevant patterns in the input and updating themselves during the training procedure. Recurrent neural networks can capture long-range dependencies in sequential data of varying lengths, such as protein or DNA sequences.

The patent publication WO 2018/006152 A1 (Deep Genomics Incorporated [CA]) published on 11 January 2018 describes systems and methods for determining molecular phenotypes from biological sequences using such convolutional neural networks.

Therefore, an opportunity arises to use deep neural networks for variant classification.

### SUMMARY OF THE INVENTION

The invention is defined in the appended claims.

### BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings.
**FIG. 1** illustrates an environment in which the variant classifier operates according to one implementation.
**FIG. 2** illustrates an example input sequence with a variant flanked by upstream and downstream bases.
**FIG. 3** shows the one-hot encoding scheme used to encode the input sequence.
**FIG. 4** shows one implementation of a metadata correlator that correlates each unclassified variant with respective values of mutation characteristics, read mapping statistics, and occurrence frequency.
**FIG. 5A** highlights some examples of context metadata features correlated with the variant.
**FIG. 5B** highlights some examples of sequencing metadata features correlated with the variant.
**FIG. 5C** highlights some examples of functional metadata features correlated with the variant.
**FIG. 5D** highlights some examples of population metadata features correlated with the variant.
**FIG. 5E** highlights one example of an ethnicity metadata feature correlated with the variant.
**FIG. 6** shows an architectural example of variant classification performed by the variant classifier.
**FIG. 7** shows an algorithmic example of variant classification performed by the variant classifier.
**FIG. 8** depicts one implementation of training the variant classifier according to a transfer learning strategy, followed by evaluation and testing of the trained variant classifier.
**FIG. 9** shows performance results of the variant caller (also referred to herein as Sojourner) on exonic data. These results, quantified by sensitivity and specificity, establish Sojourner's advantages and superiority over a non-deep neural network classifier.
**FIG. 10** shows the improvement in false positive rate using Sojourner versus the non-deep neural network classifier when classifying variants over exons.
**FIG. 11** shows the mean absolute tumor mutational burden (TMB) error using Sojourner versus the non-deep neural network classifier when classifying variants over exons.
**FIG. 12** shows the improvement in mean absolute TMB error using Sojourner versus the non-deep neural network classifier when classifying variants over exons.
**FIG. 13** shows performance results of Sojourner on CDS (coding DNA sequence) data. These results, quantified by sensitivity and specificity, establish Sojourner's advantages and superiority over the non-deep neural network classifier.
**FIG. 14** shows similar false positive rate using Sojourner versus the non-deep neural network classifier when classifying variants over coding regions.
**FIG. 15** shows the mean absolute TMB error using Sojourner versus the non-deep neural network classifier when classifying variants over coding regions.
**FIG. 16** shows similar mean absolute TMB error using Sojourner versus the non-deep neural network classifier when classifying variants over exons.
**FIG. 17** shows a computer system that can be used to implement the variant classifier.

### DETAILED DESCRIPTION

The following discussion is presented to enable any person skilled in the art to make and use the technology disclosed, and is provided in the context of a particular application and its requirements.

The discussion is organized as follows. First, an introduction describing some of the technical problems addressed by various implementations is presented, followed by an overview of the variant classifier and an explanation of terminology used throughout the discussion. Next, an example environment in which the variant classifier operates is discussed at a high-level along with a sequencing process and a variant annotation/call application. Then, various data structures fed as input to the variant classifier are discussed together with a data correlation model and some metadata samples. Next, an architectural example of variant classification performed by the variant classifier is presented, followed by an algorithmic example of the same. Then, a transfer learning strategy used to train the variant classifier is discussed in conjunction with strategies for evaluating and testing the variant classifier. Next, performance results that establish advantages and superiority of the variant classifier over a non-deep neural network classifier are presented. Lastly, various particular implementations are discussed.

### Introduction

The transformation of a normal cell into a cancer cell takes place through a sequence of discrete genetic events called somatic mutations. Tumor mutational burden (TMB) is a measurement of the number of somatic mutations per megabase of sequenced DNA and is used as a quantitative indicator for predicting response to cancer immunotherapy. Germline variant filtering is an important preprocessing step for obtaining accurate TMB assessments because only somatic variants are used for calculating TMB and germline variants are far more common than somatic variants (100-1000×).

We introduce a variant classifier that uses trained deep neural networks to predict whether a given variant is somatic or germline. Our model has two deep neural networks: a convolutional neural network (CNN) and a fully-connected neural network (FCNN). Our model receives two inputs: a DNA sequence with a variant and a set of metadata features correlated with the variant.

The first input to the model is the DNA sequence. We regard the DNA sequence as an image with multiple channels that numerically encode the four types of nucleotide bases, A, C, G, and T. The DNA sequence, spanning the variant, is one-hot encoded to conserve the position-specific information of each individual base in the sequence.

The convolutional neural network receives the one-hot encoded DNA sequence because it is capable of preserving the spatial locality relationships within the sequence. The convolutional neural network processes the DNA sequence through multiple convolution layers and produces one or more intermediate convolved features. The convolution layers utilize convolution filters to detect features within the DNA sequence. The convolution filters act as motif detectors that scan the DNA sequence for low-level motif features and produce signals of different strengths depending on the underlying sequence patterns. The convolution filters are automatically learned after training on thousands and millions of training examples of somatic and germline variants.

The second input to the model is the set of metadata features correlated with the variant. The metadata features represent the variant's mutation characteristics, read mapping statistics, and occurrence frequency. Examples of mutation characteristics are variant type, amino acid impact, evolutionary conservation, and clinical significance. Examples of read mapping statistics are variant allele frequency, read depth, and base call quality score. Examples of occurrence frequency are allele frequencies in sequenced populations and ethnic sub-populations. Some of the metadata features are encoded using categorical data such as one-hot or Boolean values, while others are encoded using continuous data such as percentage and probability values. The metadata features lack locality relationships because they are correlated only with the variant. This makes them suitable for processing by the fully-connected neural network.

First, a feature sequence is derived by concatenating the metadata features with the intermediate convolved features. The fully-connected neural network then processes the feature sequence through multiple fully-connected layers. The densely connected neurons of the fully-connected layers detect high-level features encoded in the feature sequence. Finally, a classification layer of the fully-connected neural network outputs probabilities for the variant being somatic, germline, or noise. Having the noise category improves classification along the somatic and germline categories.

Pairs of batch normalization and rectified linear unit nonlinearity are interspersed between the convolutional layers and the fully-connected layers to enhance learning rates and reduce overfitting. The model is pre-trained on somatic and germline variants from The Cancer Genome Atlas (TCGA) dataset and then fine-tuned on the TruSight Tumor (TST) dataset according a transfer learning strategy. Results demonstrate the effectiveness and efficiency of our model on validation data held-out from the TST dataset. These results, quantified by sensitivity and specificity, establish advantages and superiority of our model over traditional classifiers.

### Terminology

As used herein, the following terms have the meanings indicated.

Some portions of this application, particularly the drawings, refer to the variant classifier as "Sojourner".

A base refers to a nucleotide base or nucleotide, A (adenine), C (cytosine), T (thymine), or G (guanine).

The term "chromosome" refers to the heredity-bearing gene carrier of a living cell, which is derived from chromatin strands comprising DNA and protein components (especially histones). The conventional internationally recognized individual human genome chromosome numbering system is employed herein.

The term "site" refers to a unique position (e.g., chromosome ID, chromosome position and orientation) on a reference genome. In some implementations, a site may be a residue, a sequence tag, or a segment's position on a sequence. The term "locus" may be used to refer to the specific location of a nucleic acid sequence or polymorphism on a reference chromosome.

The term "sample" herein refers to a sample, typically derived from a biological fluid, cell, tissue, organ, or organism containing a nucleic acid or a mixture of nucleic acids containing at least one nucleic acid sequence that is to be sequenced and/or phased. Such samples include, but are not limited to sputum/oral fluid, amniotic fluid, blood, a blood fraction, fine needle biopsy samples (e.g., surgical biopsy, fine needle biopsy, etc.), urine, peritoneal fluid, pleural fluid, tissue explant, organ culture and any other tissue or cell preparation, or fraction or derivative thereof or isolated therefrom. Although the sample is often taken from a human subject (e.g., patient), samples can be taken from any organism having chromosomes, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc.

The term "sequence" includes or represents a strand of nucleotides coupled to each other. The nucleotides may be based on DNA or RNA. It should be understood that one sequence may include multiple sub-sequences. For example, a single sequence (e.g., of a PCR amplicon) may have 350 nucleotides. The sample read may include multiple sub-sequences within these 350 nucleotides. For instance, the sample read may include first and second flanking subsequences having, for example, 20-50 nucleotides. The first and second flanking sub-sequences may be located on either side of a repetitive segment having a corresponding sub-sequence (e.g., 40-100 nucleotides). Each of the flanking sub-sequences may include (or include portions of) a primer sub-sequence (e.g., 10-30 nucleotides). For ease of reading, the term "sub-sequence" will be referred to as "sequence," but it is understood that two sequences are not necessarily separate from each other on a common strand. To differentiate the various sequences described herein, the sequences may be given different labels (e.g., target sequence, primer sequence, flanking sequence, reference sequence, and the like). Other terms, such as "allele," may be given different labels to differentiate between like objects.

The term "paired-end sequencing" refers to sequencing methods that sequence both ends of a target fragment. Paired-end sequencing may facilitate detection of genomic rearrangements and repetitive segments, as well as gene fusions and novel transcripts. Methodology for paired-end sequencing are described in PCT publication WO07010252, PCT application Serial No. PCTGB2007/003798 and US patent application publication US 2009/0088327. In one example, a series of operations may be performed as follows; (a) generate clusters of nucleic acids; (b) linearize the nucleic acids; (c) hybridize a first sequencing primer and carry out repeated cycles of extension, scanning and deblocking, as set forth above; (d) "invert" the target nucleic acids on the flow cell surface by synthesizing a complimentary copy; (e) linearize the resynthesized strand; and (f) hybridize a second sequencing primer and carry out repeated cycles of extension, scanning and deblocking, as set forth above. The inversion operation can be carried out be delivering reagents as set forth above for a single cycle of bridge amplification.

The term "reference genome" or "reference sequence" refers to any particular known genome sequence, whether partial or complete, of any organism which may be used to reference identified sequences from a subject. For example, a reference genome used for human subjects as well as many other organisms is found at the National Center for Biotechnology Information at ncbi.nlm.nih.gov. A "genome" refers to the complete genetic information of an organism or virus, expressed in nucleic acid sequences. A genome includes both the genes and the noncoding sequences of the DNA. The reference sequence may be larger than the reads that are aligned to it. For example, it may be at least about 100 times larger, or at least about 1000 times larger, or at least about 10,000 times larger, or at least about 105 times larger, or at least about 106 times larger, or at least about 107 times larger. In one example, the reference genome sequence is that of a full length human genome. In another example, the reference genome sequence is limited to a specific human chromosome such as chromosome 13. In some implementations, a reference chromosome is a chromosome sequence from human genome version hg19. Such sequences may be referred to as chromosome reference sequences, although the term reference genome is intended to cover such sequences. Other examples of reference sequences include genomes of other species, as well as chromosomes, sub-chromosomal regions (such as strands), etc., of any species. In various implementations, the reference genome is a consensus sequence or other combination derived from multiple individuals. However, in certain applications, the reference sequence may be taken from a particular individual.

The term "read" refer to a collection of sequence data that describes a fragment of a nucleotide sample or reference. The term "read" may refer to a sample read and/or a reference read. Typically, though not necessarily, a read represents a short sequence of contiguous base pairs in the sample or reference. The read may be represented symbolically by the base pair sequence (in ATCG) of the sample or reference fragment. It may be stored in a memory device and processed as appropriate to determine whether the read matches a reference sequence or meets other criteria. A read may be obtained directly from a sequencing apparatus or indirectly from stored sequence information concerning the sample. In some cases, a read is a DNA sequence of sufficient length (e.g., at least about 25 bp) that can be used to identify a larger sequence or region, e.g., that can be aligned and specifically assigned to a chromosome or genomic region or gene.

Next-generation sequencing methods include, for example, sequencing by synthesis technology (Illumina), pyrosequencing (454), ion semiconductor technology (Ion Torrent sequencing), single-molecule real-time sequencing (Pacific Biosciences) and sequencing by ligation (SOLiD sequencing). Depending on the sequencing methods, the length of each read may vary from about 30 bp to more than 10,000 bp. For example, Illumina sequencing method using SOLiD sequencer generates nucleic acid reads of about 50 bp. For another example, Ion Torrent Sequencing generates nucleic acid reads of up to 400 bp and 454 pyrosequencing generates nucleic acid reads of about 700 bp. For yet another example, single-molecule real-time sequencing methods may generate reads of 10,000 bp to 15,000 bp. Therefore, in certain implementations, the nucleic acid sequence reads have a length of 30-100 bp, 50-200 bp, or 50-400 bp.

The terms "sample read", "sample sequence" or "sample fragment" refer to sequence data for a genomic sequence of interest from a sample. For example, the sample read comprises sequence data from a PCR amplicon having a forward and reverse primer sequence. The sequence data can be obtained from any select sequence methodology. The sample read can be, for example, from a sequencing-by-synthesis (SBS) reaction, a sequencing-by-ligation reaction, or any other suitable sequencing methodology for which it is desired to determine the length and/or identity of a repetitive element. The sample read can be a consensus (e.g., averaged or weighted) sequence derived from multiple sample reads. In certain implementations, providing a reference sequence comprises identifying a locus-of-interest based upon the primer sequence of the PCR amplicon.

The term "raw fragment" refers to sequence data for a portion of a genomic sequence of interest that at least partially overlaps a designated position or secondary position of interest within a sample read or sample fragment. Non-limiting examples of raw fragments include a duplex stitched fragment, a simplex stitched fragment, a duplex un-stitched fragment and a simplex un-stitched fragment. The term "raw" is used to indicate that the raw fragment includes sequence data having some relation to the sequence data in a sample read, regardless of whether the raw fragment exhibits a supporting variant that corresponds to and authenticates or confirms a potential variant in a sample read. The term "raw fragment" does not indicate that the fragment necessarily includes a supporting variant that validates a variant call in a sample read. For example, when a sample read is determined by a variant call application to exhibit a first variant, the variant call application may determine that one or more raw fragments lack a corresponding type of "supporting" variant that may otherwise be expected to occur given the variant in the sample read.

The terms "mapping", "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain implementations, to a particular location in the reference sequence. In some cases, alignment simply tells whether or not a read is a member of a particular reference sequence (i.e., whether the read is present or absent in the reference sequence). For example, the alignment of a read to the reference sequence for human chromosome 13 will tell whether the read is present in the reference sequence for chromosome 13. A tool that provides this information may be called a set membership tester. In some cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

The term "indel" refers to the insertion and/or the deletion of bases in the DNA of an organism. A micro-indel represents an indel that results in a net change of 1 to 50 nucleotides. In coding regions of the genome, unless the length of an indel is a multiple of 3, it will produce a frameshift mutation. Indels can be contrasted with point mutations. An indel inserts and deletes nucleotides from a sequence, while a point mutation is a form of substitution that replaces one of the nucleotides without changing the overall number in the DNA. Indels can also be contrasted with a Tandem Base Mutation (TBM), which may be defined as substitution at adjacent nucleotides (primarily substitutions at two adjacent nucleotides, but substitutions at three adjacent nucleotides have been observed.

The term "variant" refers to a nucleic acid sequence that is different from a nucleic acid reference. Typical nucleic acid sequence variant includes without limitation single nucleotide polymorphism (SNP), short deletion and insertion polymorphisms (Indel), copy number variation (CNV), microsatellite markers or short tandem repeats and structural variation. Somatic variant calling is the effort to identify variants present at low frequency in the DNA sample. Somatic variant calling is of interest in the context of cancer treatment. Cancer is caused by an accumulation of mutations in DNA. A DNA sample from a tumor is generally heterogeneous, including some normal cells, some cells at an early stage of cancer progression (with fewer mutations), and some late-stage cells (with more mutations). Because of this heterogeneity, when sequencing a tumor (e.g., from an FFPE sample), somatic mutations will often appear at a low frequency. For example, a SNV might be seen in only 10% of the reads covering a given base. A variant that is to be classified as somatic or germline by the variant classifier is also referred to herein as the "variant under test".

The term "noise" refers to a mistaken variant call resulting from one or more errors in the sequencing process and/or in the variant call application.

The term "variant frequency" represents the relative frequency of an allele (variant of a gene) at a particular locus in a population, expressed as a fraction or percentage. For example, the fraction or percentage may be the fraction of all chromosomes in the population that carry that allele. By way of example, sample variant frequency represents the relative frequency of an allele/variant at a particular locus/position along a genomic sequence of interest over a "population" corresponding to the number of reads and/or samples obtained for the genomic sequence of interest from an individual. As another example, a baseline variant frequency represents the relative frequency of an allele/variant at a particular locus/position along one or more baseline genomic sequences where the "population" corresponding to the number of reads and/or samples obtained for the one or more baseline genomic sequences from a population of normal individuals.

The term "variant allele frequency (VAF)" refers to the percentage of sequenced reads observed matching the variant divided by the overall coverage at the target position. VAF is a measure of the proportion of sequenced reads carrying the variant.

The terms "position", "designated position", and "locus" refer to a location or coordinate of one or more nucleotides within a sequence of nucleotides. The terms "position", "designated position", and "locus" also refer to a location or coordinate of one or more base pairs in a sequence of nucleotides.

The term "haplotype" refers to a combination of alleles at adjacent sites on a chromosome that are inherited together. A haplotype may be one locus, several loci, or an entire chromosome depending on the number of recombination events that have occurred between a given set of loci, if any occurred.

The term "threshold" herein refers to a numeric or non-numeric value that is used as a cutoff to characterize a sample, a nucleic acid, or portion thereof (e.g., a read). A threshold may be varied based upon empirical analysis. The threshold may be compared to a measured or calculated value to determine whether the source giving rise to such value suggests should be classified in a particular manner. Threshold values can be identified empirically or analytically. The choice of a threshold is dependent on the level of confidence that the user wishes to have to make the classification. The threshold may be chosen for a particular purpose (e.g., to balance sensitivity and selectivity). As used herein, the term "threshold" indicates a point at which a course of analysis may be changed and/or a point at which an action may be triggered. A threshold is not required to be a predetermined number. Instead, the threshold may be, for instance, a function that is based on a plurality of factors. The threshold may be adaptive to the circumstances. Moreover, a threshold may indicate an upper limit, a lower limit, or a range between limits.

In some implementations, a metric or score that is based on sequencing data may be compared to the threshold. As used herein, the terms "metric" or "score" may include values or results that were determined from the sequencing data or may include functions that are based on the values or results that were determined from the sequencing data. Like a threshold, the metric or score may be adaptive to the circumstances. For instance, the metric or score may be a normalized value. As an example of a score or metric, one or more implementations may use count scores when analyzing the data. A count score may be based on number of sample reads. The sample reads may have undergone one or more filtering stages such that the sample reads have at least one common characteristic or quality. For example, each of the sample reads that are used to determine a count score may have been aligned with a reference sequence or may be assigned as a potential allele. The number of sample reads having a common characteristic may be counted to determine a read count. Count scores may be based on the read count. In some implementations, the count score may be a value that is equal to the read count. In other implementations, the count score may be based on the read count and other information. For example, a count score may be based on the read count for a particular allele of a genetic locus and a total number of reads for the genetic locus. In some implementations, the count score may be based on the read count and previously-obtained data for the genetic locus. In some implementations, the count scores may be normalized scores between predetermined values. The count score may also be a function of read counts from other loci of a sample or a function of read counts from other samples that were concurrently run with the sample-of-interest. For instance, the count score may be a function of the read count of a particular allele and the read counts of other loci in the sample and/or the read counts from other samples. As one example, the read counts from other loci and/or the read counts from other samples may be used to normalize the count score for the particular allele.

The terms "coverage" or "fragment coverage" refer to a count or other measure of a number of sample reads for the same fragment of a sequence. A read count may represent a count of the number of reads that cover a corresponding fragment. Alternatively, the coverage may be determined by multiplying the read count by a designated factor that is based on historical knowledge, knowledge of the sample, knowledge of the locus, etc.

The term "read depth" (conventionally a number followed by "×") refers to the number of sequenced reads with overlapping alignment at the target position. This is often expressed as an average or percentage exceeding a cutoff over a set of intervals (such as exons, genes, or panels). For example, a clinical report might say that a panel average coverage is 1,105× with 98% of targeted bases covered >100×.

The terms "base call quality score" or "Q score" refer to a PHRED-scaled probability ranging from 0-20 inversely proportional to the probability that a single sequenced base is correct. For example, a T base call with Q of 20 is considered likely correct with a confidence P-value of 0.01. Any base call with Q<20 should be considered low quality, and any variant identified where a substantial proportion of sequenced reads supporting the variant are of low quality should be considered potentially false positive.

The terms "variant reads" or "variant read number" refer to the number of sequenced reads supporting the presence of the variant.

### Environment

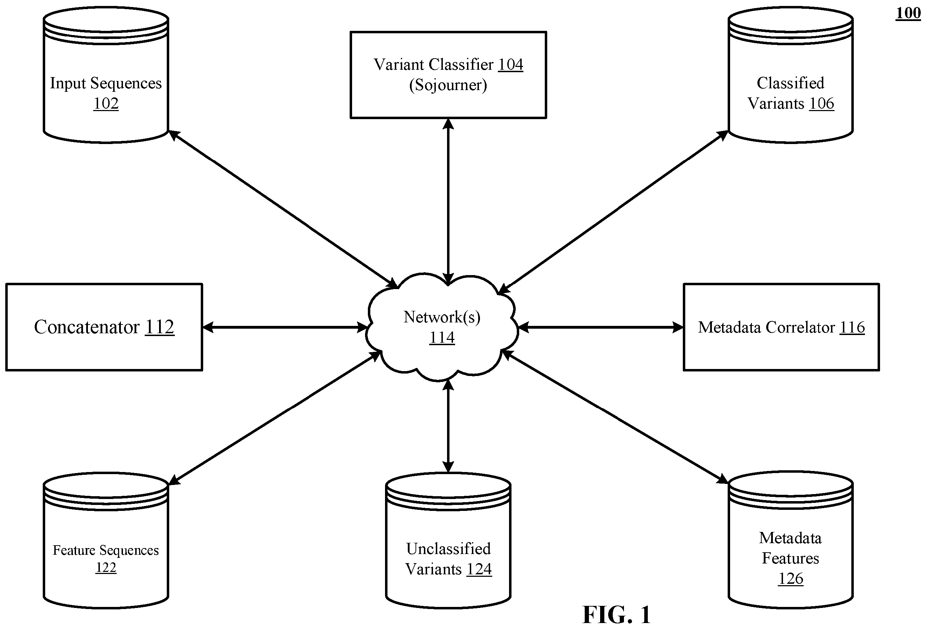

We describe a system and various implementations for variant classification using a so-called Sojourner variant classifier. The system and processes are described with reference to **FIG. 1****.** Because **FIG. 1** is an architectural diagram, certain details are intentionally omitted to improve the clarity of the description. The discussion of **FIG. 1** is organized as follows. First, the modules of the figure are introduced, followed by their interconnections. Then, the use of the modules is described in greater detail.

**FIG. 1** illustrates an environment **100** in which the variant classifier **104** operates according to one implementation. The environment **100** includes the following processing engines: variant classifier **104,** concatenator **112,** and metadata correlator **116.** The environment **100** also includes the following databases: unclassified variants **124,** input sequences **102,** metadata features **126,** and feature sequences **122.**

The processing engines and databases of **FIG. 1****,** designated as modules, can be implemented in hardware or software, and need not be divided up in precisely the same blocks as shown in **FIG. 1****.** Some of the modules can also be implemented on different processors, computers, or servers, or spread among a number of different processors, computers, or servers. In addition, it will be appreciated that some of the modules can be combined, operated in parallel or in a different sequence than that shown in **FIG. 1** without affecting the functions achieved. The modules in **FIG. 1** can also be thought of as flowchart steps in a method. A module also need not necessarily have all its code disposed contiguously in memory; some parts of the code can be separated from other parts of the code with code from other modules or other functions disposed in between.

The interconnections of the modules of environment **100** are now described. The network(s) **114** couples the processing engines and the databases, all in communication with each other (indicated by solid double-arrowed lines). The actual communication path can be point-to-point over public and/or private networks. The communications can occur over a variety of networks, e.g., private networks, VPN, MPLS circuit, or Internet, and can use appropriate application programming interfaces (APIs) and data interchange formats, e.g., Representational State Transfer (REST), JavaScript Object Notation (JSON), Extensible Markup Language (XML), Simple Object Access Protocol (SOAP), Java Message Service (JMS), and/or Java Platform Module System. All of the communications can be encrypted. The communication is generally over a network such as the LAN (local area network), WAN (wide area network), telephone network (Public Switched Telephone Network (PSTN), Session Initiation Protocol (SIP), wireless network, point-to-point network, star network, token ring network, hub network, Internet, inclusive of the mobile Internet, via protocols such as EDGE, 3G, 4G LTE, Wi-Fi, and WiMAX. Additionally, a variety of authorization and authentication techniques, such as username/password, Open Authorization (OAuth), Kerberos, SecureID, digital certificates and more, can be used to secure the communications.

### Sequencing Process

Implementations set forth herein may be applicable to analyzing nucleic acid sequences to identify sequence variations. Implementations may be used to analyze potential variants/alleles of a genetic position/locus and determine a genotype of the genetic locus or, in other words, provide a genotype call for the locus. By way of example, nucleic acid sequences may be analyzed in accordance with the methods and systems described in US Patent Application Publication No. 2016/0085910 and US Patent Application Publication No. 2013/02961175.

In one implementation, a sequencing process includes receiving a sample that includes or is suspected of including nucleic acids, such as DNA. The sample may be from a known or unknown source, such as an animal (e.g., human), plant, bacteria, or fungus. The sample may be taken directly from the source. For instance, blood or saliva may be taken directly from an individual. Alternatively, the sample may not be obtained directly from the source. Then, one or more processors direct the system to prepare the sample for sequencing. The preparation may include removing extraneous material and/or isolating certain material (e.g., DNA). The biological sample may be prepared to include features for a particular assay. For example, the biological sample may be prepared for sequencing-by-synthesis (SBS). In certain implementations, the preparing may include amplification of certain regions of a genome. For instance, the preparing may include amplifying predetermined genetic loci that are known to include STRs and/or SNPs. The genetic loci may be amplified using predetermined primer sequences.

Next, the one or more processors direct the system to sequence the sample. The sequencing may be performed through a variety of known sequencing protocols. In particular implementations, the sequencing includes SBS. In SBS, a plurality of fluorescently-labeled nucleotides are used to sequence a plurality of clusters of amplified DNA (possibly millions of clusters) present on the surface of an optical substrate (e.g., a surface that at least partially defines a channel in a flow cell). The flow cells may contain nucleic acid samples for sequencing where the flow cells are placed within the appropriate flow cell holders.

The nucleic acids can be prepared such that they comprise a known primer sequence that is adjacent to an unknown target sequence. To initiate the first SBS sequencing cycle, one or more differently labeled nucleotides, and DNA polymerase, etc., can be flowed into/through the flow cell by a fluid flow subsystem. Either a single type of nucleotide can be added at a time, or the nucleotides used in the sequencing procedure can be specially designed to possess a reversible termination property, thus allowing each cycle of the sequencing reaction to occur simultaneously in the presence of several types of labeled nucleotides (e.g., A, C, T, G). The nucleotides can include detectable label moieties such as fluorophores. Where the four nucleotides are mixed together, the polymerase is able to select the correct base to incorporate and each sequence is extended by a single base. Non-incorporated nucleotides can be washed away by flowing a wash solution through the flow cell. One or more lasers may excite the nucleic acids and induce fluorescence. The fluorescence emitted from the nucleic acids is based upon the fluorophores of the incorporated base, and different fluorophores may emit different wavelengths of emission light. A deblocking reagent can be added to the flow cell to remove reversible terminator groups from the DNA strands that were extended and detected. The deblocking reagent can then be washed away by flowing a wash solution through the flow cell. The flow cell is then ready for a further cycle of sequencing starting with introduction of a labeled nucleotide as set forth above. The fluidic and detection operations can be repeated several times to complete a sequencing run. Example sequencing methods are described, for example, in Bentley et al., Nature 456:53-59 (2008), International Publication No. WO 04/018497; U.S. Pat. No. 7,057,026; International Publication No. WO 91/06678; International Publication No. WO 07/123744; U.S. Pat. No. 7,329,492; U.S. Patent No. 7,211,414; U.S. Patent No. 7,315,019; U.S. Patent No. 7,405,281, and U.S. Patent Application Publication No. 2008/0108082.

In some implementations, nucleic acids can be attached to a surface and amplified prior to or during sequencing. For example, amplification can be carried out using bridge amplification to form nucleic acid clusters on a surface. Useful bridge amplification methods are described, for example, in U.S. Patent No. 5,641,658; U.S. Patent Application Publication No. 2002/0055100; U.S. Patent No. 7,115,400; U.S. Patent Application Publication No. 2004/0096853; U.S. Patent Application Publication No. 2004/0002090; U.S. Patent Application Publication No. 2007/0128624; and U.S. Patent Application Publication No. 2008/0009420. Another useful method for amplifying nucleic acids on a surface is rolling circle amplification (RCA), for example, as described in Lizardi et al., Nat. Genet. 19:225-232 (1998) and U.S. Patent Application Publication No. 2007/0099208 A1.

One example SBS protocol exploits modified nucleotides having removable 3' blocks, for example, as described in International Publication No. WO 04/018497, U.S. Patent Application Publication No. 2007/0166705A1, and U.S. Patent No. 7,057,026. For example, repeated cycles of SBS reagents can be delivered to a flow cell having target nucleic acids attached thereto, for example, as a result of the bridge amplification protocol. The nucleic acid clusters can be converted to single stranded form using a linearization solution. The linearization solution can contain, for example, a restriction endonuclease capable of cleaving one strand of each cluster. Other methods of cleavage can be used as an alternative to restriction enzymes or nicking enzymes, including inter alia chemical cleavage (e.g., cleavage of a diol linkage with periodate), cleavage of abasic sites by cleavage with endonuclease (for example 'USER', as supplied by NEB, Ipswich, Mass., USA, part number M5505S), by exposure to heat or alkali, cleavage of ribonucleotides incorporated into amplification products otherwise comprised of deoxyribonucleotides, photochemical cleavage or cleavage of a peptide linker. After the linearization operation a sequencing primer can be delivered to the flow cell under conditions for hybridization of the sequencing primer to the target nucleic acids that are to be sequenced.

A flow cell can then be contacted with an SBS extension reagent having modified nucleotides with removable 3' blocks and fluorescent labels under conditions to extend a primer hybridized to each target nucleic acid by a single nucleotide addition. Only a single nucleotide is added to each primer because once the modified nucleotide has been incorporated into the growing polynucleotide chain complementary to the region of the template being sequenced there is no free 3'-OH group available to direct further sequence extension and therefore the polymerase cannot add further nucleotides. The SBS extension reagent can be removed and replaced with scan reagent containing components that protect the sample under excitation with radiation. Example components for scan reagent are described in U.S. Patent Application Publication No. 2008/0280773 A1 and U.S. Patent Application No. 13/018,255. The extended nucleic acids can then be fluorescently detected in the presence of scan reagent. Once the fluorescence has been detected, the 3' block may be removed using a deblock reagent that is appropriate to the blocking group used. Example deblock reagents that are useful for respective blocking groups are described in WO004018497, US 2007/0166705A1 and U.S. Patent No. 7,057,026. The deblock reagent can be washed away leaving target nucleic acids hybridized to extended primers having 3'-OH groups that are now competent for addition of a further nucleotide. Accordingly the cycles of adding extension reagent, scan reagent, and deblock reagent, with optional washes between one or more of the operations, can be repeated until a desired sequence is obtained. The above cycles can be carried out using a single extension reagent delivery operation per cycle when each of the modified nucleotides has a different label attached thereto, known to correspond to the particular base. The different labels facilitate discrimination between the nucleotides added during each incorporation operation. Alternatively, each cycle can include separate operations of extension reagent delivery followed by separate operations of scan reagent delivery and detection, in which case two or more of the nucleotides can have the same label and can be distinguished based on the known order of delivery.

Although the sequencing operation has been discussed above with respect to a particular SBS protocol, it will be understood that other protocols for sequencing any of a variety of other molecular analyses can be carried out as desired.

Then, the one or more processors of the system receive the sequencing data for subsequent analysis. The sequencing data may be formatted in various manners, such as in a .BAM file. The sequencing data may include, for example, a number of sample reads. The sequencing data may include a plurality of sample reads that have corresponding sample sequences of the nucleotides. Although only one sample read is discussed, it should be understood that the sequencing data may include, for example, hundreds, thousands, hundreds of thousands, or millions of sample reads. Different sample reads may have different numbers of nucleotides. For example, a sample read may range between 10 nucleotides to about 500 nucleotides or more. The sample reads may span the entire genome of the source(s). As one example, the sample reads are directed toward predetermined genetic loci, such as those genetic loci having suspected STRs or suspected SNPs.

Each sample read may include a sequence of nucleotides, which may be referred to as a sample sequence, sample fragment or a target sequence. The sample sequence may include, for example, primer sequences, flanking sequences, and a target sequence. The number of nucleotides within the sample sequence may include 30, 40, 50, 60, 70, 80, 90, 100 or more. In some implementations, one or more the sample reads (or sample sequences) includes at least 150 nucleotides, 200 nucleotides, 300 nucleotides, 400 nucleotides, 500 nucleotides, or more. In some implementations, the sample reads may include more than 1000 nucleotides, 2000 nucleotides, or more. The sample reads (or the sample sequences) may include primer sequences at one or both ends.

Next, the one or more processors analyze the sequencing data to obtain potential variant call(s) and a sample variant frequency of the sample variant call(s). The operation may also be referred to as a variant call application or variant caller. Thus, the variant caller identifies or detects variants and the variant classifier classifies the detected variants as somatic or germline. Alternative variant callers may be utilized in accordance with implementations herein, wherein different variant callers may be used based on the type of sequencing operation being performed, based on features of the sample that are of interest and the like. One non-limiting example of a variant call application, such as the Pisces^{™} application by Illumina Inc. (San Diego, CA) hosted at https://github.com/Illumina/Pisces and described in the article Dunn, Tamsen & Berry, Gwenn & Emig-Agius, Dorothea & Jiang, Yu & Iyer, Anita & Udar, Nitin & Strömberg, Michael. (2017). Pisces: An Accurate and Versatile Single Sample Somatic and Germline Variant Caller. 595-595. 10.1145/3107411.3108203.

Such a variant call application can comprise four sequentially executed modules:
(1) Pisces Read Stitcher: Reduces noise by stitching paired reads in a BAM (read one and read two of the same molecule) into consensus reads. The output is a stitched BAM.
(2) Pisces Variant Caller: Calls small SNVs, insertions and deletions. Pisces includes a variant-collapsing algorithm to coalesce variants broken up by read boundaries, basic filtering algorithms, and a simple Poisson-based variant confidence-scoring algorithm. The output is a VCF.
(3) Pisces Variant Quality Recalibrator (VQR): In the event that the variant calls overwhelmingly follow a pattern associated with thermal damage or FFPE deamination, the VQR step will downgrade the variant Q score of the suspect variant calls. The output is an adjusted VCF.
(4) Pisces Variant Phaser (Scylla): Uses a read-backed greedy clustering method to assemble small variants into complex alleles from clonal subpopulations. This allows for the more accurate determination of functional consequence by downstream tools. The output is an adjusted VCF.

Additionally or alternatively, the operation may utilize the variant call application Strelka^{™} application by Illumina Inc. hosted at https://github.com/Illumina/strelka and described in the article T Saunders, Christopher & Wong, Wendy & Swamy, Sajani & Becq, Jennifer & J Murray, Lisa & Cheetham, Keira. (2012). Strelka: Accurate somatic small-variant calling from sequenced tumor-normal sample pairs. Bioinformatics (Oxford, England). 28. 1811-7. 10.1093/bioinformatics/bts271. Furthermore, additionally or alternatively, the operation may utilize the variant call application Strelka2^{™} application by Illumina Inc. hosted at https://github.com/Illumina/strelka and described in the article Kim, S., Scheffler, K., Halpern, A.L., Bekritsky, M.A., Noh, E., Källberg, M., Chen, X., Beyter, D., Krusche, P., and Saunders, C.T. (2017). Strelka2: Fast and accurate variant calling for clinical sequencing applications. Moreover, additionally or alternatively, the operation may utilize a variant annotation/call tool, such as the Nirvana^{™} application by Illumina Inc. hosted at https://github.com/Illumina/Nirvana/wiki and described in the article Stromberg, Michael & Roy, Rajat & Lajugie, Julien & Jiang, Yu & Li, Haochen & Margulies, Elliott. (2017). Nirvana: Clinical Grade Variant Annotator. 596-596. 10.1145/3107411.3108204.

Such a variant annotation/call tool can apply different algorithmic techniques such as those disclosed in Nirvana:
a. Identifying all overlapping transcripts with Interval Array: For functional annotation, we can identify all transcripts overlapping a variant and an interval tree can be used. However, since a set of intervals can be static, we were able to further optimize it to an Interval Array. An interval tree returns all overlapping transcripts in O(min(n,k lg n)) time, where n is the number of intervals in the tree and k is the number of overlapping intervals. In practice, since k is really small compared to n for most variants, the effective runtime on interval tree would be O(k lg n) . We improved to O(lg n + k ) by creating an interval array where all intervals are stored in a sorted array so that we only need to find the first overlapping interval and then enumerate through the remaining (k-1).
b. CNVs/SVs (Yu): annotations for Copy Number Variation and Structural Variants can be provided. Similar to the annotation of small variants, transcripts overlapping with the SV and also previously reported structural variants can be annotated in online databases. Unlike the small variants, not all overlapping transcripts need be annotated, since too many transcripts will be overlapped with a large SVs. Instead, all overlapping transcripts can be annotated that belong to a partial overlapping gene. Specifically, for these transcripts, the impacted introns, exons and the consequences caused by the structural variants can be reported. An option to allow output all overlapping transcripts is available, but the basic information for these transcripts can be reported, such as gene symbol, flag whether it is canonical overlap or partial overlapped with the transcripts. For each SV/CNV, it is also of interest to know if these variants have been studied and their frequencies in different populations. Hence, we reported overlapping SVs in external databases, such as 1000 genomes, DGV and ClinGen. To avoid using an arbitrary cutoff to determine which SV is overlapped, instead all overlapping transcripts can be used and the reciprocal overlap can be calculated, i.e. the overlapping length divided by the minimum of the length of these two SVs.
c. Reporting supplementary annotations : Supplementary annotations are of two types: small and structural variants (SVs). SVs can be modeled as intervals and use the interval array discussed above to identify overlapping SVs. Small variants are modeled as points and matched by position and (optionally) allele. As such, they are searched using a binary-search-like algorithm. Since the supplementary annotation database can be quite large, a much smaller index is created to map chromosome positions to file locations where the supplementary annotation resides. The index is a sorted array of objects (made up of chromosome position and file location) that can be binary searched using position. To keep the index size small, multiple positions (up to a certain max count) are compressed to one object that stores the values for the first position and only deltas for subsequent positions. Since we use Binary search, the runtime is O(lg n) , where n is the number of items in the database.
d. VEP cache files
e. Transcript Database : The Transcript Cache (cache) and Supplementary database (SAdb) files are serialized dump of data objects such as transcripts and supplementary annotations. We use Ensembl VEP cache as our data source for cache. To create the cache, all transcripts are inserted in an interval array and the final state of the array is stored in the cache files. Thus, during annotation, we only need to load a pre-computed interval array and perform searches on it. Since the cache is loaded up in memory and searching is very fast (described above), finding overlapping transcripts is extremely quick in Nirvana (profiled to less than 1% of total runtime?).
f. Supplementary Database : The data sources for SAdb are listed under supplementary material. The SAdb for small variants is produced by a k -way merge of all data sources such that each object in the database (identified by reference name and position) holds all relevant supplementary annotations. Issues encountered during parsing data source files have been documented in detail in Nirvana's home page. To limit memory usage, only the SA index is loaded up in memory. This index allows a quick lookup of the file location for a supplementary annotation. However, since the data has to be fetched from disk, adding supplementary annotation has been identified as Nirvana's largest bottleneck (profiled at ~30% of total runtime.)
g. Consequence and Sequence Ontology : Nirvana's functional annotation (when provided) follows the Sequence Ontology (SO) (http://www.sequenceontology.org/ ) guidelines. On occasions, we had the opportunity to identify issues in the current SO and collaborate with the SO team to improve the state of annotation.

Such a variant annotation tool can include pre-processing. For example, Nirvana included a large number of annotations from External data sources, like ExAC, EVS, 1000 Genomes project, dbSNP, ClinVar, Cosmic, DGV and ClinGen. To make full use of these databases, we have to sanitize the information from them. We implemented different strategy to deal with different conflicts that exist from different data sources. For example, in case of multiple dbSNP entries for the same position and alternate allele, we join all ids into a comma separated list of ids; if there are multiple entries with different CAF values for the same allele, we use the first CAF value. For conflicting ExAC and EVS entries, we consider the number of sample counts and the entry with higher sample count is used. In 1000 Genome Projects, we removed the allele frequency of the conflicting allele. Another issue is inaccurate information. We mainly extracted the allele frequencies information from 1000 Genome Projects, however, we noticed that for GRCh38, the allele frequency reported in the info field did not exclude samples with genotype not available, leading to deflated frequencies for variants which are not available for all samples. To guarantee the accuracy of our annotation, we use all of the individual level genotype to compute the true allele frequencies. As we know, the same variants can have different representations based on different alignments. To make sure we can accurately report the information for already identified variants, we have to preprocess the variants from different resources to make them have consistent representation. For all external data sources, we trimmed alleles to remove duplicated nucleotides in both reference allele and alternative allele. For ClinVar, we directly parsed the xml file we performed a five-prime alignment for all variants, which is often used in vcf file. Different databases can contain the same set of information. To avoid unnecessary duplicates, we removed some duplicated information. For example, we removed variants in DGV which has data source as 1000 genome projects, since we already reported these variants in 1000 genomes with more detailed information.

In accordance with at least some implementations, the variant call application provides calls for low frequency variants, germline calling and the like. As non-limiting example, the variant call application may run on tumor-only samples and/or tumor-normal paired samples. The variant call application may search for single nucleotide variations (SNV), multiple nucleotide variations (MNV), indels and the like. The variant call application identifies variants, while filtering for mismatches due to sequencing or sample preparation errors. For each variant, the variant caller identifies the reference sequence, a position of the variant, and the potential variant sequence(s) (e.g., A to C SNV, or AG to A deletion). The variant call application identifies the sample sequence (or sample fragment), a reference sequence/fragment, and a variant call as an indication that a variant is present. The variant call application may identify raw fragments, and output a designation of the raw fragments, a count of the number of raw fragments that verify the potential variant call, the position within the raw fragment at which a supporting variant occurred and other relevant information. Non-limiting examples of raw fragments include a duplex stitched fragment, a simplex stitched fragment, a duplex un-stitched fragment and a simplex un- stitched fragment.

The variant call application may output the calls in various formats, such as in a .VCF or .GVCF file. By way of example only, the variant call application may be included in a MiSeqReporter pipeline (e.g., when implemented on the MiSeq^{®} sequencer instrument). Optionally, the application may be implemented with various workflows. The analysis may include a single protocol or a combination of protocols that analyze the sample reads in a designated manner to obtain desired information.

Then, the one or more processors perform a validation operation in connection with the potential variant call. The validation operation may be based on a quality score, and/or a hierarchy of tiered tests, as explained hereafter. When the validation operation authenticates or verifies that the potential variant call, the validation operation passes the variant call information (from the variant call application) to the sample report generator. Alternatively, when the validation operation invalidates or disqualifies the potential variant call, the validation operation passes a corresponding indication (e.g., a negative indicator, a no call indicator, an in-valid call indicator) to the sample report generator. The validation operation also may pass a confidence score related to a degree of confidence that the variant call is correct or the in-valid call designation is correct.

Next, the one or more processors generate and store a sample report. The sample report may include, for example, information regarding a plurality of genetic loci with respect to the sample. For example, for each genetic locus of a predetermined set of genetic loci, the sample report may at least one of provide a genotype call; indicate that a genotype call cannot be made; provide a confidence score on a certainty of the genotype call; or indicate potential problems with an assay regarding one or more genetic loci. The sample report may also indicate a gender of an individual that provided a sample and/or indicate that the sample include multiple sources. As used herein, a "sample report" may include digital data (e.g., a data file) of a genetic locus or predetermined set of genetic locus and/or a printed report of the genetic locus or the set of genetic loci. Thus, generating or providing may include creating a data file and/or printing the sample report, or displaying the sample report.

The sample report may indicate that a variant call was determined, but was not validated. When a variant call is determined invalid, the sample report may indicate additional information regarding the basis for the determination to not validate the variant call. For example, the additional information in the report may include a description of the raw fragments and an extent (e.g., a count) to which the raw fragments support or contradicted the variant call. Additionally or alternatively, the additional information in the report may include the quality score obtained in accordance with implementations described herein.

### Variant Call Application

Implementations disclosed herein include analyzing sequencing data to identify potential variant calls. Variant calling may be performed upon stored data for a previously performed sequencing operation. Additionally or alternatively, it may be performed in real time while a sequencing operation is being performed. Each of the sample reads is assigned to corresponding genetic loci. The sample reads may be assigned to corresponding genetic loci based on the sequence of the nucleotides of the sample read or, in other words, the order of nucleotides within the sample read (e.g., A, C, G, T). Based on this analysis, the sample read may be designated as including a possible variant/allele of a particular genetic locus. The sample read may be collected (or aggregated or binned) with other sample reads that have been designated as including possible variants/alleles of the genetic locus. The assigning operation may also be referred to as a calling operation in which the sample read is identified as being possibly associated with a particular genetic position/locus. The sample reads may be analyzed to locate one or more identifying sequences (e.g., primer sequences) of nucleotides that differentiate the sample read from other sample reads. More specifically, the identifying sequence(s) may identify the sample read from other sample reads as being associated with a particular genetic locus.

The assigning operation may include analyzing the series of n nucleotides of the identifying sequence to determine if the series of n nucleotides of the identifying sequence effectively matches with one or more of the select sequences. In particular implementations, the assigning operation may include analyzing the first n nucleotides of the sample sequence to determine if the first n nucleotides of the sample sequence effectively matches with one or more of the select sequences. The number n may have a variety of values, which may be programmed into the protocol or entered by a user. For example, the number n may be defined as the number of nucleotides of the shortest select sequence within the database. The number n may be a predetermined number. The predetermined number may be, for example, 10, 11, 12, 13, 14, 15, 16, 17, 18, 19, 20, 21, 22, 23, 24, 25, 26, 27, 28, 29, or 30 nucleotides. However, fewer or more nucleotides may be used in other implementations. The number n may also be selected by an individual, such as a user of the system. The number n may be based on one or more conditions. For instance, the number n may be defined as the number of nucleotides of the shortest primer sequence within the database or a designated number, whichever is the smaller number. In some implementations, a minimum value for n may be used, such as 15, such that any primer sequence that is less than 15 nucleotides may be designated as an exception.

In some cases, the series of n nucleotides of an identifying sequence may not precisely match the nucleotides of the select sequence. Nonetheless, the identifying sequence may effectively match the select sequence if the identifying sequence is nearly identical to the select sequence. For example, the sample read may be called for a genetic locus if the series of n nucleotides (e.g., the first n nucleotides) of the identifying sequence match a select sequence with no more than a designated number of mismatches (e.g., 3) and/or a designated number of shifts (e.g., 2). Rules may be established such that each mismatch or shift may count as a difference between the sample read and the primer sequence. If the number of differences is less than a designated number, then the sample read may be called for the corresponding genetic locus (i.e., assigned to the corresponding genetic locus). In some implementations, a matching score may be determined that is based on the number of differences between the identifying sequence of the sample read and the select sequence associated with a genetic locus. If the matching score passes a designated matching threshold, then the genetic locus that corresponds to the select sequence may be designated as a potential locus for the sample read. In some implementations, subsequent analysis may be performed to determine whether the sample read is called for the genetic locus.

If the sample read effectively matches one of the select sequences in the database (i.e., exactly matches or nearly matches as described above), then the sample read is assigned or designated to the genetic locus that correlates to the select sequence. This may be referred to as locus calling or provisional-locus calling, wherein the sample read is called for the genetic locus that correlates to the select sequence. However, as discussed above, a sample read may be called for more than one genetic locus. In such implementations, further analysis may be performed to call or assign the sample read for only one of the potential genetic loci. In some implementations, the sample read that is compared to the database of reference sequences is the first read from paired- end sequencing. When performing paired-end sequencing, a second read (representing a raw fragment) is obtained that correlates to the sample read. After assigning, the subsequent analysis that is performed with the assigned reads may be based on the type of genetic locus that has been called for the assigned read.

Next, the sample reads are analyzed to identify potential variant calls. Among other things, the results of the analysis identify the potential variant call, a sample variant frequency, a reference sequence and a position within the genomic sequence of interest at which the variant occurred. For example, if a genetic locus is known for including SNPs, then the assigned reads that have been called for the genetic locus may undergo analysis to identify the SNPs of the assigned reads. If the genetic locus is known for including polymorphic repetitive DNA elements, then the assigned reads may be analyzed to identify or characterize the polymorphic repetitive DNA elements within the sample reads. In some implementations, if an assigned read effectively matches with an STR locus and an SNP locus, a warning or flag may be assigned to the sample read. The sample read may be designated as both an STR locus and an SNP locus. The analyzing may include aligning the assigned reads in accordance with an alignment protocol to determine sequences and/or lengths of the assigned reads. The alignment protocol may include the method described in International Patent Application No. PCT/US2013/030867 (Publication No. WO 2014/142831), filed on March 15, 2013.

Then, the one or more processors analyze raw fragments to determine whether supporting variants exist at corresponding positions within the raw fragments. Various types of raw fragments may be identified. For example, the variant caller may identify a type of raw fragment that exhibits a variant that validates the original variant call. For example, the type of raw fragment may represent a duplex stitched fragment, a simplex stitched fragment, a duplex un-stitched fragment or a simplex un-stitched fragment. Optionally other raw fragments may be identified instead of or in addition to the foregoing examples. In connection with identifying each type of raw fragment, the variant caller also identifies the position, within the raw fragment, at which the supporting variant occurred, as well as a count of the number of raw fragments that exhibited the supporting variant. For example, the variant caller may output an indication that 10 reads of raw fragments were identified to represent duplex stitched fragments having a supporting variant at a particular position X. The variant caller may also output indication that five reads of raw fragments were identified to represent simplex un-stitched fragments having a supporting variant at a particular position Y. The variant caller may also output a number of raw fragments that corresponded to reference sequences and thus did not include a supporting variant that would otherwise provide evidence validating the potential variant call at the genomic sequence of interest.

Next, a count is maintained of the raw fragments that include supporting variants, as well as the position at which the supporting variant occurred. Additionally or alternatively, a count may be maintained of the raw fragments that did not include supporting variants at the position of interest (relative to the position of the potential variant call in the sample read or sample fragment). Additionally or alternatively, a count may be maintained of raw fragments that correspond to a reference sequence and do not authenticate or confirm the potential variant call. The information determined is output to the variant call validation application, including a count and type of the raw fragments that support the potential variant call, positions of the supporting variance in the raw fragments, a count of the raw fragments that do not support the potential variant call and the like.

When a potential variant call is identified, the process outputs an indicating of the potential variant call, the variant sequence, the variant position and a reference sequence associated therewith. The variant call is designated to represent a "potential" variant as errors may cause the call process to identify a false variant. In accordance with implementations herein, the potential variant call is analyzed to reduce and eliminate false variants or false positives. Additionally or alternatively, the process analyzes one or more raw fragments associated with a sample read and outputs a corresponding variant call associated with the raw fragments.

### Data Structures

Database **124** includes variants that have not yet been classified as somatic or germline. These variants are detected by the sequencing process and the variant annotation/call applications described above. The DNA segments, spanning the variants, can be derived from tumor samples or tumor-normal pair samples. The variants can be single-nucleotide polymorphisms (SNPs), insertions, or deletions. The variants can also be crawled from publicly available databases such as The Cancer Genome Atlas (TCGA), International Cancer Genome Consortium (ICGC), database of short genetic variants (dbSNP), Catalog of Somatic Mutations in Cancer (COSMIC), 1000 Genomes Project (1000Genomes), Exome Aggregation Consortium (ExAC), and Exome Variant Server (EVS). Prior to being added to the database **124,** the variants can be filtered based on criteria such as cancer association, cancer type (e.g., lung adenocarcinoma (LUAD), variant allele frequency (VAF), and coding region (exonic/intronic).

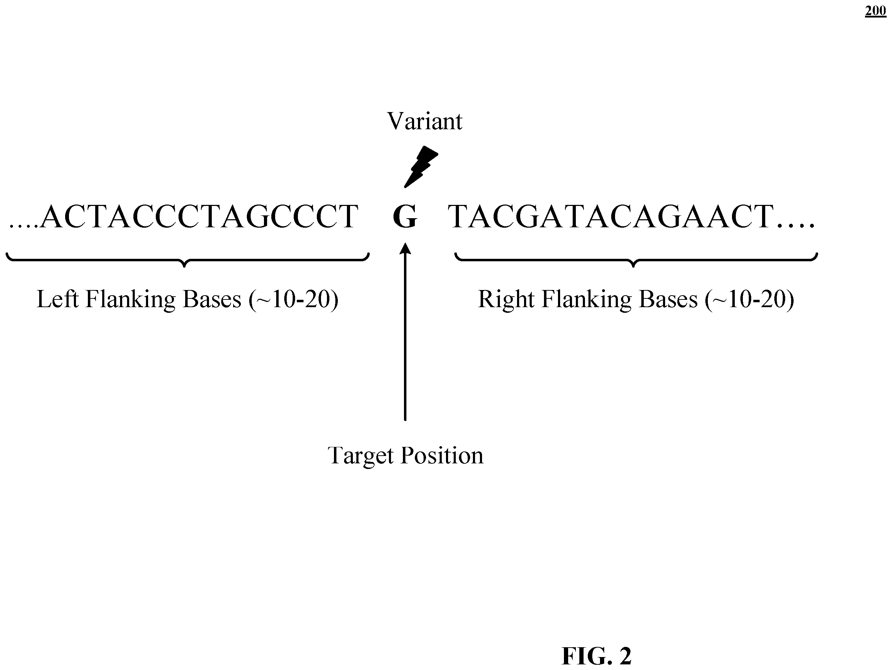

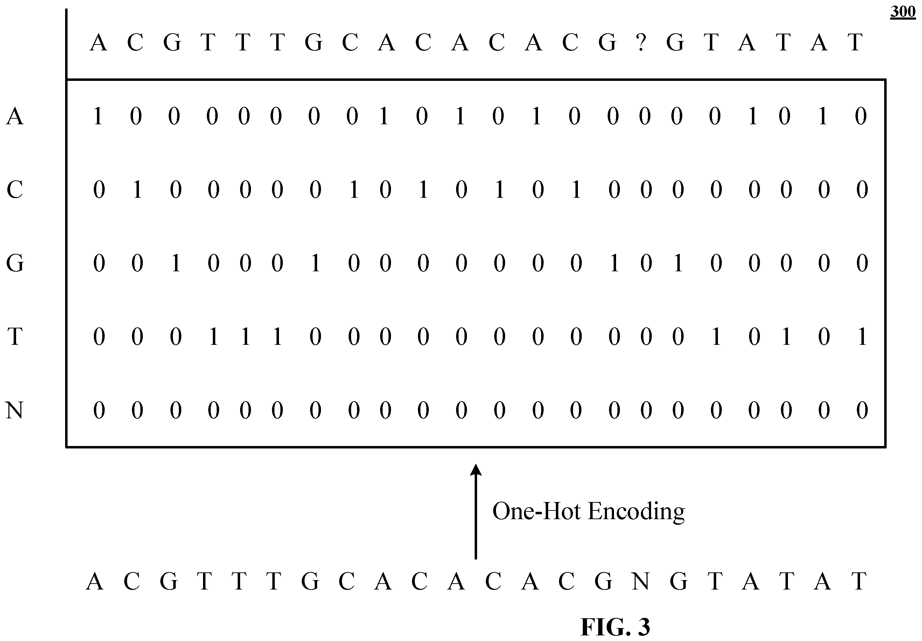

Database **102** includes input sequences that are one-hot encodings of DNA segments containing the variants. **FIG. 2** illustrates an example input sequence **200** with a variant at a target position flanked by upstream (left) and downstream (right) bases. **FIG. 3** shows the one-hot encoding scheme **300** used to encode the input sequence. The following is an example of a one-hot encoding scheme (A, G, C, T, N) that is used to encode the DNA segments: A = (1 0 0 0 0), G = (0 1 0 0 0), C = (0 0 1 0 0), T = (0 0 0 1 0), and N = (0 0 0 0 1). Each input sequence includes at least one variant, preferably located at the center (target position) of the sequence. An input sequence can be 21 bases long, with the variant flanked by 10 downstream and upstream bases, or it can also be 41 bases long, with the variant flanked by 20 downstream and upstream bases. It will be appreciated that input sequences of varying lengths can be constructed. In contrast to being based on naturally occurring DNA, the input sequences can be simulated by selecting a variant from the database **124** and flanking it with randomly generated downstream and upstream bases.

### Data Correlation Model

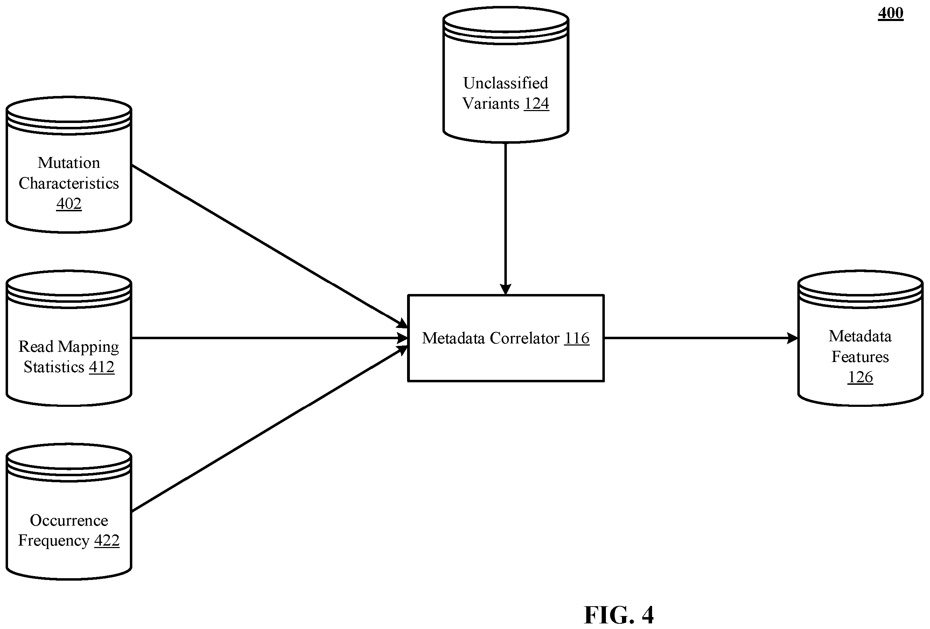

**FIG. 4** shows one implementation of the metadata correlator **116** that correlates each unclassified variant in the database **124** with respective values of mutation characteristics, read mapping statistics, and occurrence frequency. In implementation, the metadata correlator **116** includes the Nirvana^{™} clinical-grade variant annotation application discussed above along with one or more ethnicity detection applications. The metadata correlator **116** encodes the correlations in so-called metadata features that are stored in the database **126.** Correlation **400** is performed on a variant-by-variant basis and includes identifying attributes of a particular variant in the databases **402, 412, and 422** and associating/linking/appending the found attributes with or to the variant.

Database **402** includes mutation characteristics of the variant, such as whether the variant is a SNP, an insertion, or deletion; whether the variant is nonsynonymous or not; what was the base(s) in the reference sequence that the variant mutated; what is the clinical significance of the variant as determined from clinical tests (e.g., clinical effect, drug sensitivity, and histocompatibility); evolutionary conservation of the variant position across multiple species (e.g., mammals, birds), what is the ethnic makeup of the individual that provided the tumor sample associated with the variant, and what is the functional impact of the variant on resulting proteins. Database **402** represents one or more publically available databases and tools such as ClinVar, Polymorphism Phenotyping (PolyPhen), Sorting Intolerant from Tolerant (SIFT), and phylop. Database **402** can also be populated by data from the sequencing process and the variant annotation/call applications described above (e.g., from the .BAM file, the .VCF or .GVCF file, the sample report, and/or the count). For example, whether the variant is a SNP, an insertion, or deletion and whether the variant is nonsynonymous or not is determined from the .VCF file, according to one implementation.

Database **412** includes read mapping statistics of the variant, such as variant allele frequency (VAF), read depth, base call quality score (Q score), variant reads (variant read number), variant quality scores (QUAL), mapping quality scores, and Fisher strand bias. Database **412** is populated by data from the sequencing process and the variant annotation/call applications described above (e.g., from the .BAM file, the .VCF or .GVCF file, the sample report, and/or the count).

Database **422** includes occurrence frequency of the variant, such as allele frequencies of the variant in sequenced populations, allele frequencies of the variant in ethnic sub-populations stratified from sequenced populations, frequency of the variant sequenced cancerous tumors. Database **422** represents one or more publically available databases such as database of short genetic variants (dbSNP), 1000 Genomes Project (1000Genomes), Exome Aggregation Consortium (ExAC), Exome Variant Server (EVS), Genome Aggregation Database (gnomAD), and Catalog of Somatic Mutations in Cancer (COSMIC). Database **422** can also be populated by data from the sequencing process and the variant annotation/call applications described above (e.g., from the .BAM file, the .VCF or .GVCF file, the sample report, and/or the count).

### Metadata Samples

The following are two samples of metadata features A to Q produced by the metadata correlator **116.** As discussed above, some of the metadata features are encoded using categorical data such as one-hot or Boolean values, while others are encoded using continuous data such as percentage and probability values. In implementations, only a subset of the metadata features are provided as input to the variant caller. For example, in some implementations, the chromosome feature, the reference sequence feature, and the coordinate position feature are not included in the metadata features that are provided as input.

### First sample:

A. Name: chromosome feature
Description: specifies the chromosome on which the DNA segment spanning the variant occurs.
Type: mutation characteristic

| | | |
|---|---|---|
| 1. | chr | chr1 |

B. Name: reference sequence feature
Description: specifies the reference sequence mutated by the variant.
Type: mutation characteristic

| | | |
|---|---|---|
| 1. | ref | C |

C. Name: coordinate position feature
Description: specifies the coordinate position of the variant on the chromosome.
Type: mutation characteristic

| | | |
|---|---|---|
| 1. | pos | 11205058 |

D. Name: alternative allele feature
Description: specifies at least one base mutated by the variant at the target position in the reference sequence.
Type: mutation characteristic

| | | |
|---|---|---|
| 1. | alt_A | -1.0 |
| 2. | alt_C | -1.0 |
| 3. | alt_G | -1.0 |
| 4. | alt_T | 1.0 |
| 5. | alt_Other | -1.0 |

E. Name: variant allele frequency feature
Description: specifies variant allele frequency (VAF) of the variant.
Type: read mapping statistic

| | | |
|---|---|---|
| 1. | VAF | 1.0 |

F. Name: read depth feature
Description: specifies read depth of the variant.
Type: read mapping statistic

| | | |
|---|---|---|
| 1. | dp | 1.07 |

G. Name: mutation type feature
Description: specifies whether the variant is a single-nucleotide variant (SNV), insertion, or deletion.
Type: mutation characteristic

| | | |
|---|---|---|
| 1. | type_snv | 1.0 |
| 2. | type_insertion | -1.0 |
| 3. | type_deletion | -1.0 |

H. Name: population frequency feature
Description: specifies allele frequencies of the variant in sequenced populations such as database of short genetic variants (dbSNP), 1000 Genomes Project (1000Genomes), Exome Aggregation Consortium (ExAC), and Exome Variant Server (EVS).
Type: occurrence frequency

| | | |
|---|---|---|
| 1. | dbsnp | 0.4525 |
| 2. | oneKg | 0.547524 |
| 3. | exac | 0 |
| 4. | evs | 0 |

I. Name: amino acid impact feature
Description: specifies whether the variant is a nonsynonymous variant that changes a codon so as to produce a new codon which codes for a different amino acid.
Type: mutation characteristic

| | | |
|---|---|---|
| 1. | nonsyn_true | -1.0 |
| 2. | nonsyn_false | 1.0 |

J. Name: evolutionary conservation feature
Description: specifies conservativeness of the variant position across multiple species, as determined from phylop.
Type: mutation characteristic

| | | |
|---|---|---|
| 1. | phylop | 0.078 |

K. Name: evolutionary conservation data availability feature
Description: specifies whether any phylop data is available.
Type: mutation characteristic

| | | |
|---|---|---|
| 1. | phylop_NA | 1 |

L. Name: clinical significance feature
Description: specifies the variant's clinical effect, drug sensitivity, and histocompatibility as determined from clinical test results submitted on ClinVar.
Type: mutation characteristic

| | | |
|---|---|---|
| 1. | clinvarSig_drug response | -1.0 |
| 2. | clinvarSig_uncertain significance | -1.0 |
| 3. | clinvarSig_likely pathogenic | -1.0 |
| 4. | clinvarSig_pathogenic | -1.0 |
| 5. | clinvarSig_not provided | -1.0 |
| 6. | clinvarSig_nan | 1.0 |
| 7. | clinvarSig_likely benign | -1.0 |
| 8. | clinvarSig_benign | -1.0 |
| 9. | clinvarSig_other | -1.0 |

M. Name: functional impact feature
Description: specifies the variant's impact on functionality of a protein resulting from an amino acid substitution caused by the variant as determined from Polymorphism Phenotyping (PolyPhen).
Type: mutation characteristic

| | | |
|---|---|---|
| 1. | polyPhen_benign | -1.0 |
| 2. | polyPhen_possibly damaging | -1.0 |
| 3. | polyPhen_nan | 1.0 |
| 4. | polyPhen_probably damaging | -1.0 |
| 5. | polyPhen_unknown | -1.0 |

N. Name: functional impact feature
Description: specifies the variant's impact on functionality of a protein resulting from an amino acid substitution caused by the variant as determined from Sorting Intolerant from Tolerant (SIFT).
Type: mutation characteristic

| | | |
|---|---|---|
| 1. | sift_tolerated | -1.0 |
| 2. | sift_deleterious - low confidence | -1.0 |
| 3. | sift_nan | 1.0 |
| 4. | sift_deleterious | -1.0 |
| 5. | sift_tolerated - low confidence | -1.0 |

O. Name: tumor frequency feature
Description: specifies frequency of the variant in sequenced cancerous tumors as determined from Catalog of Somatic Mutations in Cancer (COSMIC) database.
Type: occurrence frequency

| | | |
|---|---|---|
| 1. | CNT | 2.09217 |

P. Name: sub-population frequency feature
Description: specifies allele frequencies of the variant in ethnic sub-populations stratified from sequenced populations as determined from Genome Aggregation Database (gnomAD) database.
Type: occurrence frequency

| | | |
|---|---|---|
| 1. | gnomadExomeAf | 0.04 |
| 2. | gnomadExome_afrAf | 0.686792 |
| 3. | gnomadExome_asmrAf | 0.14098000000000002 |
| 4. | gnomadExome_easAf | 00.8134640000000001 |
| 5. | gnomadExome_finAf | 0.7214389999999999 |
| 6. | gnomadExome_nfeAf | 0.7409239999999999 |
| 7. | gnomadExome_asjAf | 0.5827749999999999 |
| 8. | gnomadExome_sasAf | 0.654254 |
| 9. | gnomadExome_othAf | 0.684902 |
| 10. | gnomadAf | 0.5688719999999999 |
| 11. | gnomad_afrAf | 0.15348399999999998 |
| 12. | gnomad_asmrAf | 0 |
| 13. | gnomad_easAf | 0.8003709999999999 |
| 14. | gnomad_finAf | 0.709336 |
| 15. | gnomad _nfeAf | 0.737876 |
| 16. | gnomad_asjAf | 0.55298 |
| 17. | gnomad_sasAf | 0 |
| 18. | gnomad_othAf | 0.673469 |

Q. Name: ethnicity prediction feature
Description: specifies likelihoods identifying ethnic makeup of the individual that provided the tumor sample associated with the variant.
Type: occurrence frequency

| | | |
|---|---|---|
| 1. | ethno_P_AFR | 4.137788205335579e-49 |
| 2. | ethno_P_AMR | 0.00484825490847577 |
| 3. | ethno_P_EAS | 2.4537058155646697e-55 |
| 4. | ethno_P_EUR | 0.9951517345697741 |
| 5. | ethno_P_SAS | 1.0521763446561e-08 |

### Second sample:

A. Name: chromosome feature
Description: specifies the chromosome on which the DNA segment spanning the variant occurs.
Type: mutation characteristic

| | | |
|---|---|---|
| 1. | chr | chr1 |

B. Name: reference sequence feature
Description: specifies the reference sequence mutated by the variant.
Type: mutation characteristic

| | | |
|---|---|---|
| 1. | ref | A |

C. Name: coordinate position feature
Description: specifies the coordinate position of the variant on the chromosome.
Type: mutation characteristic

| | | |
|---|---|---|
| 1. | pos | 2488153 |

D. Name: alternative allele feature
Description: specifies at least one base mutated by the variant at the target position in the reference sequence.
Type: mutation characteristic

| | | |
|---|---|---|
| 1. | alt_A | -1.0 |
| 2. | alt_C | -1.0 |
| 3. | alt_G | 1.0 |
| 4. | alt_T | -1.0 |
| 5. | alt_Other | -1.0 |

E. Name: variant allele frequency feature
Description: specifies variant allele frequency (VAF) of the variant.
Type: read mapping statistic

| | | |
|---|---|---|
| 1. | VAF | 0.9974 |

F. Name: read depth feature
Description: specifies read depth of the variant.
Type: read mapping statistic

| | | |
|---|---|---|
| 1. | dp | 3.82 |

G. Name: mutation type feature
Description: specifies whether the variant is a single-nucleotide variant (SNV), insertion, or deletion.
Type: mutation characteristic

| | | |
|---|---|---|
| 1. | type_snv | 1.0 |
| 2. | type_insertion | -1.0 |
| 3. | type_deletion | -1.0 |

H. Name: population frequency feature
Description: specifies allele frequencies of the variant in sequenced populations such as database of short genetic variants (dbSNP), 1000 Genomes Project (1000Genomes), Exome Aggregation Consortium (ExAC), and Exome Variant Server (EVS).
Type: occurrence frequency

| | | |
|---|---|---|
| 1. | dbsnp | 0.3852 |
| 2. | oneKg | 0.6148159999999999 |
| 3. | exac | 0 |
| 4. | evs | 0 |

I. Name: amino acid impact feature
Description: specifies whether the variant is a nonsynonymous variant that changes a codon so as to produce a new codon which codes for a different amino acid.
Type: mutation characteristic

| | | |
|---|---|---|
| 1. | nonsyn_true | 1.0 |
| 2. | nonsyn_false | -1.0 |

J. Name: evolutionary conservation feature
Description: specifies conservativeness of the variant position across multiple species, as determined from phylop.
Type: mutation characteristic

| | | |
|---|---|---|
| 1. | phylop | -0.17600000000000002 |

K. Name: evolutionary conservation data availability feature
Description: specifies whether any phylop data is available.
Type: mutation characteristic

| | | |
|---|---|---|
| 1. | phylop_NA | 1 |

L. Name: clinical significance feature
Description: specifies the variant's clinical effect, drug sensitivity, and histocompatibility as determined from clinical test results submitted on ClinVar.
Type: mutation characteristic

| | | |
|---|---|---|
| 1. | clinvarSig_drug response | -1.0 |
| 2. | clinvarSig_uncertain significance | -1.0 |
| 3. | clinvarSig_likely pathogenic | -1.0 |
| 4. | clinvarSig_pathogenic | -1.0 |
| 5. | clinvarSig_not provided | -1.0 |
| 6. | clinvarSig_nan | 1.0 |
| 7. | clinvarSig_likely benign | -1.0 |
| 8. | clinvarSig_benign | -1.0 |
| 9. | clinvarSig_other | -1.0 |

M. Name: functional impact feature
Description: specifies the variant's impact on functionality of a protein resulting from an amino acid substitution caused by the variant as determined from Polymorphism Phenotyping (PolyPhen).
Type: mutation characteristic

| | | |
|---|---|---|
| 1. | polyPhen_benign | 1.0 |
| 2. | polyPhen_possibly damaging | -1.0 |
| 3. | polyPhen_nan | -1.0 |
| 4. | polyPhen_probably damaging | -1.0 |
| 5. | polyPhen_unknown | -1.0 |

N. Name: functional impact feature
Description: specifies the variant's impact on functionality of a protein resulting from an amino acid substitution caused by the variant as determined from Sorting Intolerant from Tolerant (SIFT).
Type: mutation characteristic

| | | |
|---|---|---|
| 1. | sift_tolerated | 1.0 |
| 2. | sift_deleterious - low confidence | -1.0 |
| 3. | sift_nan | -1.0 |
| 4. | sift_deleterious | -1.0 |
| 5. | sift_tolerated - low confidence | -1.0 |

O. Name: tumor frequency feature
Description: specifies frequency of the variant in sequenced cancerous tumors as determined from Catalog of Somatic Mutations in Cancer (COSMIC) database.
Type: occurrence frequency

| | | |
|---|---|---|
| 1. | CNT | 3.46492 |

P. Name: sub-population frequency feature
Description: specifies allele frequencies of the variant in ethnic sub-populations stratified from sequenced populations as determined from Genome Aggregation Database (gnomAD) database.
Type: occurrence frequency

| | | |
|---|---|---|
| 1. | gnomadExomeAf | 0.04 |
| 2. | gnomadExome_afrAf | 0.512886 |
| 3. | gnomadExome_asmrAf | 0.727304 |
| 4. | gnomadExome_easAf | 00.48744 |
| 5. | gnomadExome_finAf | 0.48818900000000004 |
| 6. | gnomadExome_nfeAf | 0.466213 |
| 7. | gnomadExome_asjAf | 0.443545 |
| 8. | gnomadExome_sasAf | 0.633193 |
| 9. | gnomadExome_othAf | 0.499022 |
| 10. | gnomadAf | 0.5445989999999999 |
| 11. | gnomad_afrAf | 0.7156319999999999 |
| 12. | gnomad_asmrAf | 0 |
| 13. | gnomad_easAf | 0.46091800000000005 |
| 14. | gnomad_finAf | 0.48421400000000003 |
| 15. | gnomad_nfeAf | 0.473486 |
| 16. | gnomad_asjAf | 0.446667 |
| 17. | gnomad_sasAf | 0 |
| 18. | gnomad_othAf | 0.515369 |

Q. Name: ethnicity prediction feature
Description: specifies likelihoods identifying ethnic makeup of the individual that provided the tumor sample associated with the variant.
Type: occurrence frequency

| | | |
|---|---|---|
| 1. | ethno_P_ AFR | 4.137788205335579e-49 |
| 2. | ethno_P_AMR | 0.00484825490847577 |
| 3. | ethno_P_EAS | 2.4537058155646697e-55 |
| 4. | ethno_P_EUR | 0.9951517345697741 |
| 5. | ethno_P_SAS | 1.0521763446561e-08 |

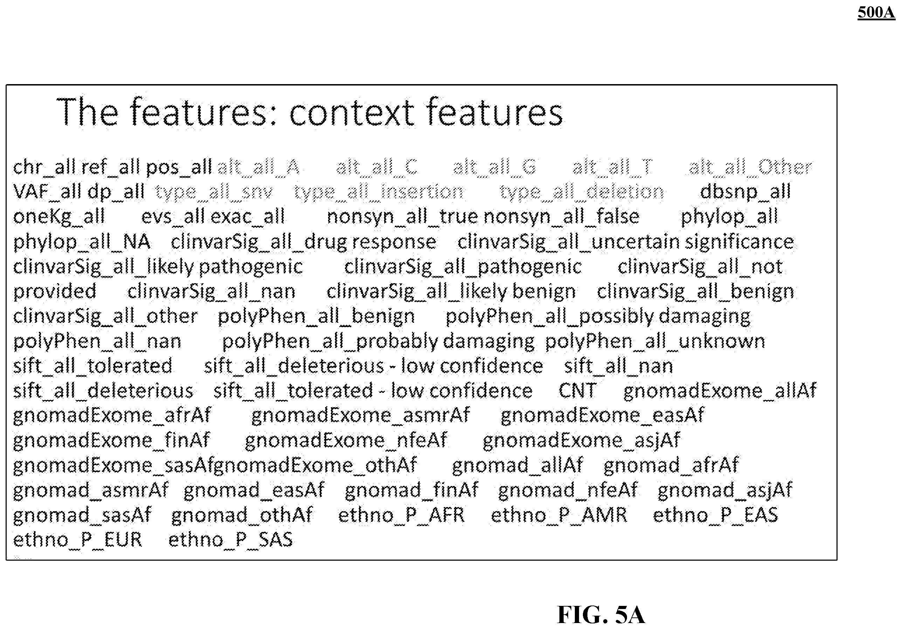

**FIG. 5A** highlights some examples of context metadata features **500A** correlated with the variant. The context metadata features **500A** collectively represent the alternative allele feature and the mutation type feature discussed above.

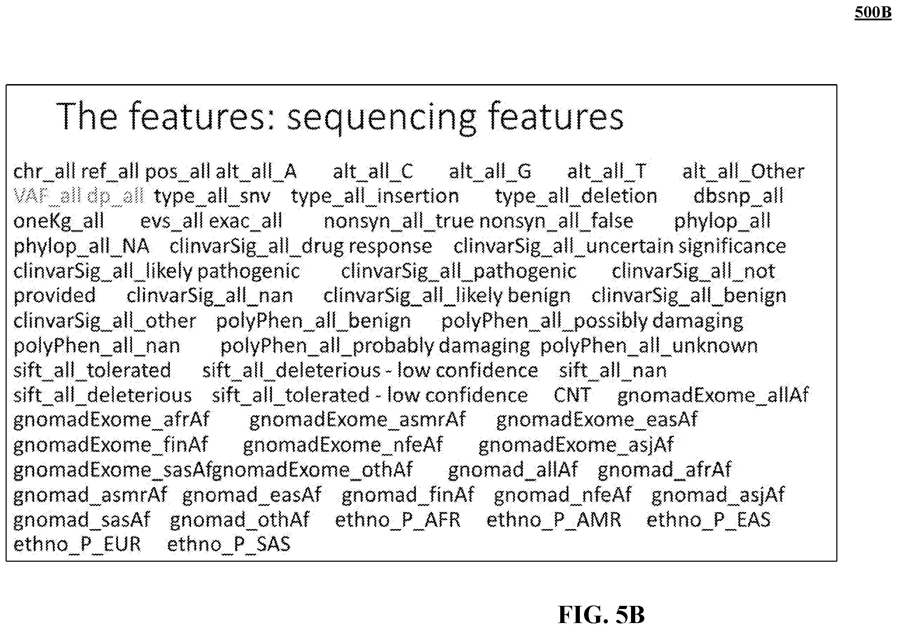

**FIG. 5B** highlights some examples of sequencing metadata features **500B** correlated with the variant. The sequencing metadata features **500B** collectively represent the variant allele frequency feature and the read depth feature discussed above.

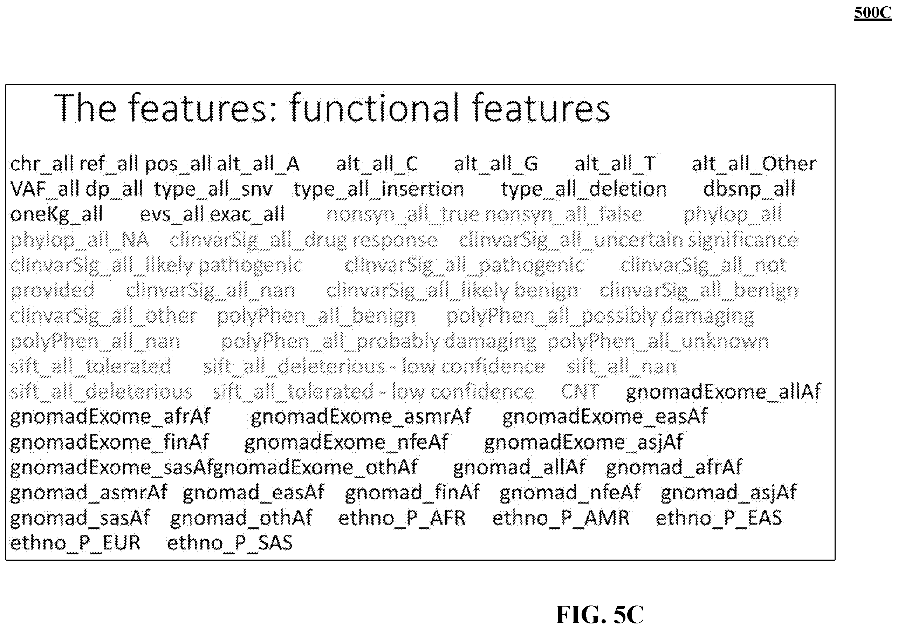

**FIG. 5C** highlights some examples of functional metadata features **500C** correlated with the variant. The functional metadata features **500C** collectively represent the amino acid impact feature, the evolutionary conservation feature, the evolutionary conservation data availability feature, the clinical significance feature, the functional impact features, and the tumor frequency feature discussed above.

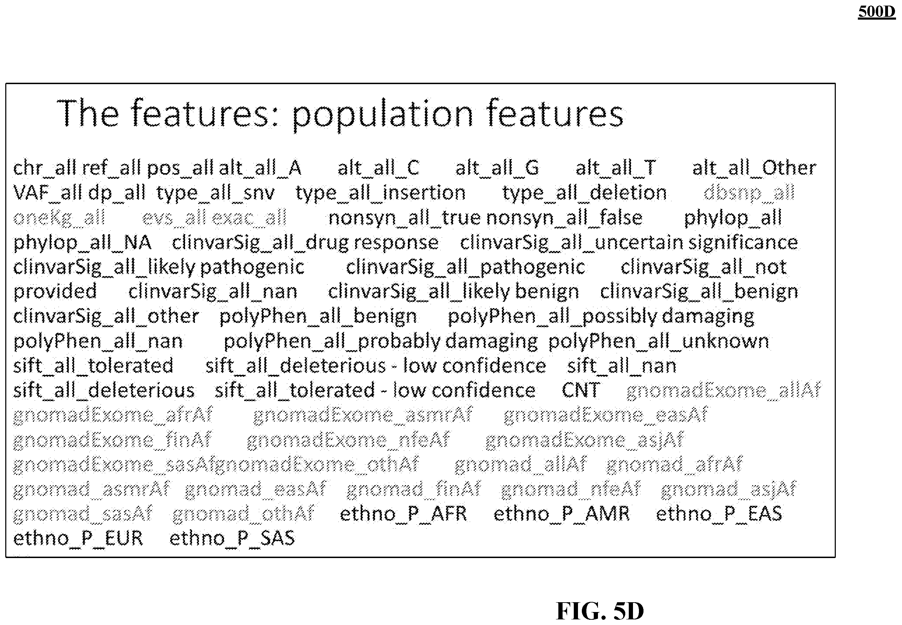

**FIG. 5D** highlights some examples of population metadata features **500D** correlated with the variant. The population metadata features **500D** collectively represent the population frequency feature and the sub-population frequency feature discussed above.

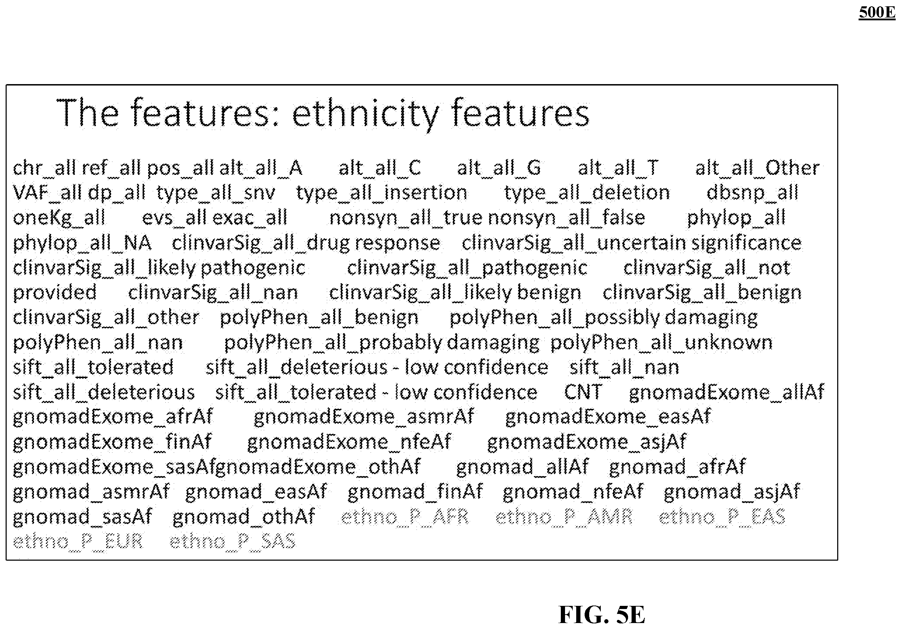

**FIG. 5E** highlights one example of an ethnicity metadata feature **500E** correlated with the variant. The ethnicity metadata feature **500E** represents the ethnicity prediction feature discussed above.

### Variant Classification

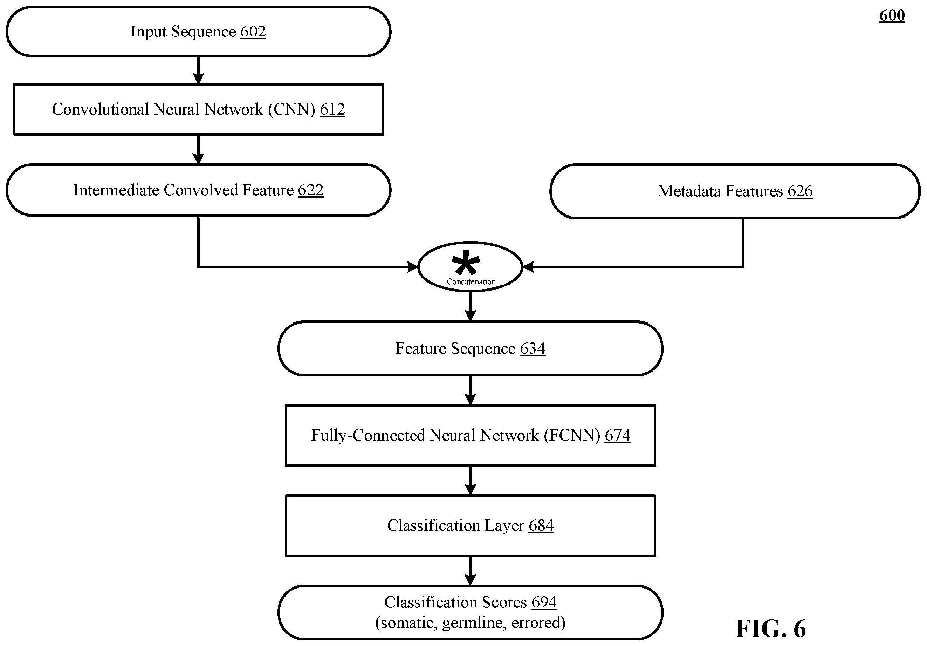

The task of the variant classifier **104** is to classify each variant in the database **124** as somatic or germline. **FIG. 6** shows an architectural example **600** of variant classification performed by the variant classifier **104.** An input sequence **602,** with a variant at a target position flanked by at least ten bases on each side, is fed as input to the convolutional neural network (CNN) **612.** Convolutional neural network **612** comprises convolution layers which perform the convolution operation between the input values and convolution filters (matrix of weights) that are learned over many gradient update iterations during the training.

Let *m* be the filter size and *W* be the matrix of weights, then a convolution layer performs a convolution of the *W* with the inputXby calculating the dot product *W* • *x + b,* where *x* is an instance of *X* and *b* is the bias. The step size by which the convolution filters slide across the input is called the stride, and the filter width *m* is called the receptive field. A same convolution filter is applied across different positions of the input, which reduces the number of weights learned. It also allows location invariant learning, i.e., if an important pattern exists in the input, the convolution filters learn it no matter where it is in the sequence. Additional details about the convolutional neural network **612** can be found in I. J. Goodfellow, D. Warde-Farley, M. Mirza, A. Courville, and Y. Bengio, "CONVOLUTIONAL NETWORKS," Deep Learning, MIT Press, 2016; J. Wu, "INTRODUCTION TO CONVOLUTIONAL NEURAL NETWORKS," Nanjing University, 2017; and N. ten DIJKE, "Convolutional Neural Networks for Regulatory Genomics," Master's Thesis, Universiteit Leiden Opleiding Informatica, 17 June 2017.

After processing the input sequence **602,** the convolutional neural network **612** produces an intermediate convolved feature **622** as output. The concatenator **112** concatenates (*) the intermediate convolved feature **622** with one or more metadata features **626** discussed above. Concatenation can occur across the row dimension or the column dimension. For example, concatenating 1x465 outputs of the intermediate convolved feature 622 and 1x49 metadata features 626 would produce a feature sequence 634 of 1x514 values. The result of the concatenation is a feature sequence **634,** which is stored in the database **122.**

The feature sequence **634** is fed as input to the fully-connected neural network (FCNN) **674.** The fully-connected neural network **674** comprises fully-connected layers - each neuron receives input from all the previous layer's neurons and sends its output to every neuron in the next layer. This contrasts with how convolutional layers work where the neurons send their output to only some of the neurons in the next layer. The neurons of the fully-connected layers are optimized over many gradient update iterations during the training. Additional details about the fully-connected neural network **674** can be found in 1. J. Goodfellow, D. Warde-Farley, M. Mirza, A. Courville, and Y. Bengio, "CONVOLUTIONAL NETWORKS," Deep Learning, MIT Press, 2016; J. Wu, "INTRODUCTION TO CONVOLUTIONAL NEURAL NETWORKS," Nanjing University, 2017; and N. ten DIJKE, "Convolutional Neural Networks for Regulatory Genomics," Master's Thesis, Universiteit Leiden Opleiding Informatica, 17 June 2017.

A classification layer 684 of the fully-connected neural network **674** outputs classification scores **694** for likelihood that the variant is a somatic variant, a germline variant, or noise. The classification layer **684** can be a softmax layer or a sigmoid layer. The number of classes and their type can be modified, depending on the implementation. As discussed above, having the noise category improves classification along the somatic and germline categories.

In other implementations, the metadata features **626** can be fed directly to the convolutional neural network **612** and encoded into the input sequence **602** or fed separately, but simultaneously with the input sequence **602** or fed separately, but before/after the input sequence **602.**

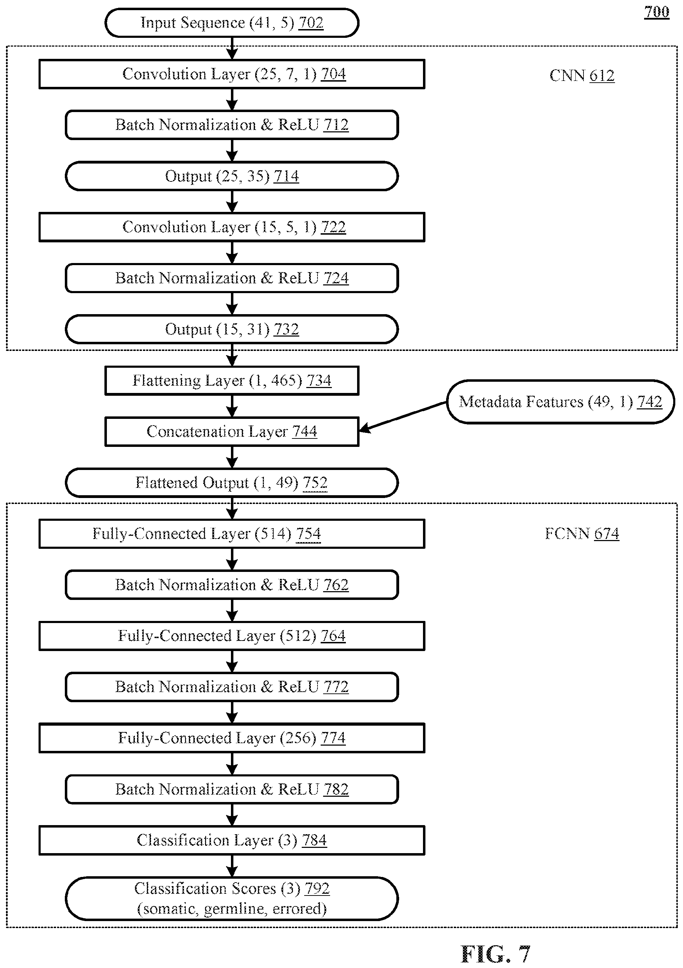

**FIG. 7** shows an algorithmic example **700** of variant classification performed by the variant classifier **104**. In the illustrated implementation, the convolution neural network (CNN) **612** has two convolution layers and the fully-connected neural network (FCNN) **674** has three fully-connected layers. In other implementations, the variant classifier **104,** and its convolution neural network **612** and fully-connected neural network **674,** can have additional, fewer, or different parameters and hyperparameters. Some examples of parameters are number of convolution layers, number of batch normalization and ReLU layers, number of fully-connected layers, number of convolution filters in respective convolution layers, number of neurons in respective fully-connected layers, number of outputs produced by the final classification layer, and residual connectivity. Some examples of hyperparameters are window size of the convolution filters, stride length of the convolution filters, padding, and dilation. In the discussion below, the term "layer" refers to an algorithm implemented in code as a software logic or module. Some examples of layers can be found in Keras^{™} documentation available at https://keras.io/layers/about-keras-layers/, the complete subject matter of which is expressly incorporated herein by reference in its entirety.

A one-hot encoded input sequence **702** is fed to a first convolution layer **704** of the convolutional neural network (CNN) **612**. The dimensionality of the input sequence **702** is 41, 5, where 41 represents the 41 bases in the input sequence **702** with a particular variant at a center target position flanked by 20 bases on each side, and 5 represents the 5 channels A, T, C, G, N used to encode the input sequence **702** and illustrated in **FIG. 3****.**

The first convolution layer **704** has 25 filters, each of which convolves over the input sequence **702** with a window size of 7 and stride length of 1. The convolution is followed by batch normalization and ReLU nonlinearity layers **712.** What results is an output (feature map) **714** of dimensionality 25, 35. Output **714** can be regarded as the first intermediate convolved feature.

Output **714** is fed as input to a second convolution layer **722** of the convolutional neural network **612.** The second convolution layer **722** has 15 filters, each of which convolves over the output **714** with a window size of 5 and stride length of 1. The convolution is followed by batch normalization and ReLU nonlinearity layers **724.** What results is an output (feature map) **732** of dimensionality 15, 31. Output **732** can be regarded as the second intennediate convolved feature and also the final output of the convolutional neural network **612.**

In order to concatenate the output **732** with the metadata features **742** and also to allow downstream processing by the fully-connected neural network (FCNN) **674**, the output **732** is flattened by a flattening layer **734.** Flattening includes vectorizing the output **732** to have either one row or one column. That is, by way of example, converting the output **732** of dimensionality 15, 31 into a flattened vector of dimensionality 1, 465 (1 row and 15x31 = 465 columns).

The metadata features **742,** correlated with the particular variant, have a dimensionality of 49, l. A concatenation layer **744** concatenates the metadata features **742** with the flattened vector derived from the output **732.** What results is a flattened metadata feature output **752** of dimensionality 1, 49. Output **752** can be regarded as the feature sequence. Concatenation of the flattened vector **732** and the flattened output **752** produces a vector 1, 514.

The output **752** is then fed as input to the fully-connected neural network (FCNN) **674.** The fully-connected neural network **674** has three fully-connected layers **754, 764,** and **774,** each succeeded by pairs **762**, **772,** and **782** of batch normalization and ReLU nonlinearity layers. The first fully-connected layer **754** has 512 neurons, which are fully connected to 512 neurons in the second fully-connected layer **764.** The 514 neurons in the second fully-connected layer **764** are fully connected to 256 neurons in the third fully-connected layer **774.**

The classification layer **784** (e.g., softmax) has 3 neurons which output the 3 classification scores or probabilities **792** for the particular variant being somatic, germline, or noise.

In other implementations, the metadata features **742** can be fed directly to the convolutional neural network **612** and encoded into the input sequence **702** or fed separately, but simultaneously with the input sequence **702** or fed separately, but before/after the input sequence **702.**

### Transfer Learning

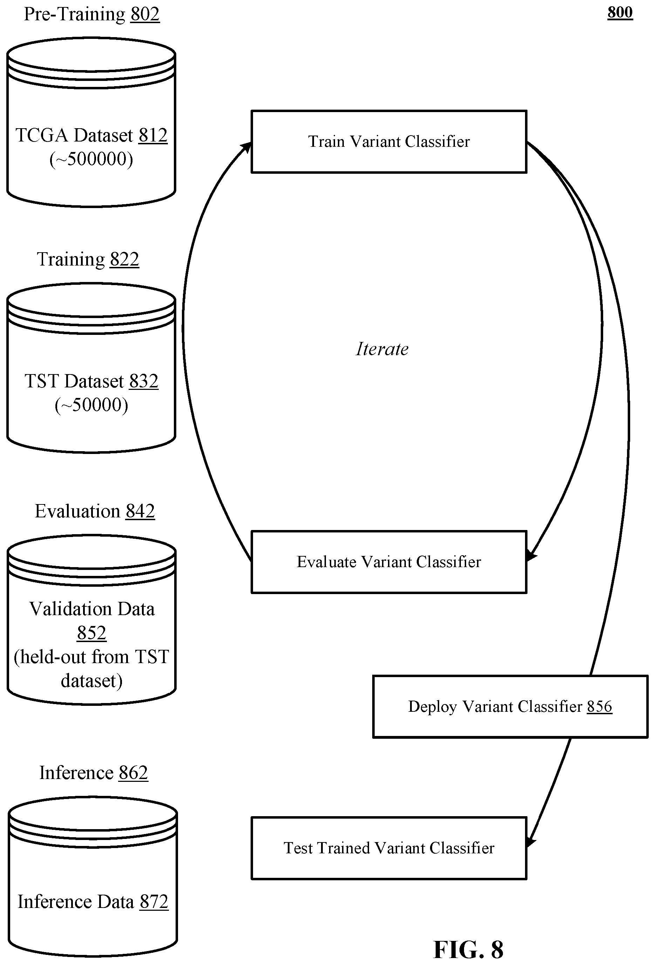

**FIG. 8** depicts one implementation of training the variant classifier **104** according to a transfer learning strategy **800**, followed by evaluation and testing of the trained variant classifier **104.** Transfer learning strategy **800** involves pre-training **802** the variant classifier **104** on a base dataset **812** (e.g., TCGA) and task (variant classification), and then repurposing or transferring the learned weights (filters, neurons) of the convolutional neural network (CNN) **612** and the fully-connected neural network **674** for training **822** on a target dataset **832** (e.g., TST) and task (variant classification). This process works well because the TCGA dataset **812** and the TST dataset **832** share common features.

Evaluation **842** includes iteratively checking the variant classification performance of the variant classifier **104** on validation data **852** held-out from the TST dataset **862.** After a convergence condition has met (e.g., meeting a certain benchmark like F-measure or minimizing error below a threshold), the trained variant classifier **104** is deployed for inference or testing **862.** Deployment **856** can include hosting the trained variant classifier **104** on a cloud-based environment like Illumina's BaseSpace^{™} for use by the research community, making the trained classifier **104** runnable on a memory chip or GPU for incorporation in mobile computing devices, and/or making the variant classifier **104** available for download from the web. During inference **862,** the trained variant classifier **104** can receive input sequences in the form of inference data **872** and perform variant classification as discussed above.

### Performance Results

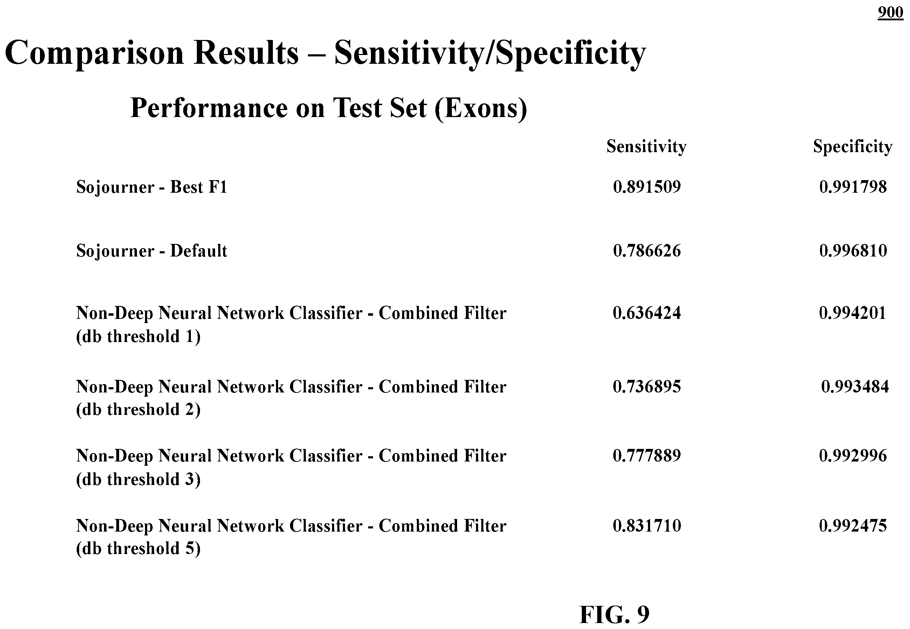

**FIG. 9** shows performance results **900** of the variant caller (also referred to herein as Sojourner) on exonic data. These results, quantified by sensitivity and specificity, establish Sojourner's advantages and superiority over a non-deep neural network classifier.

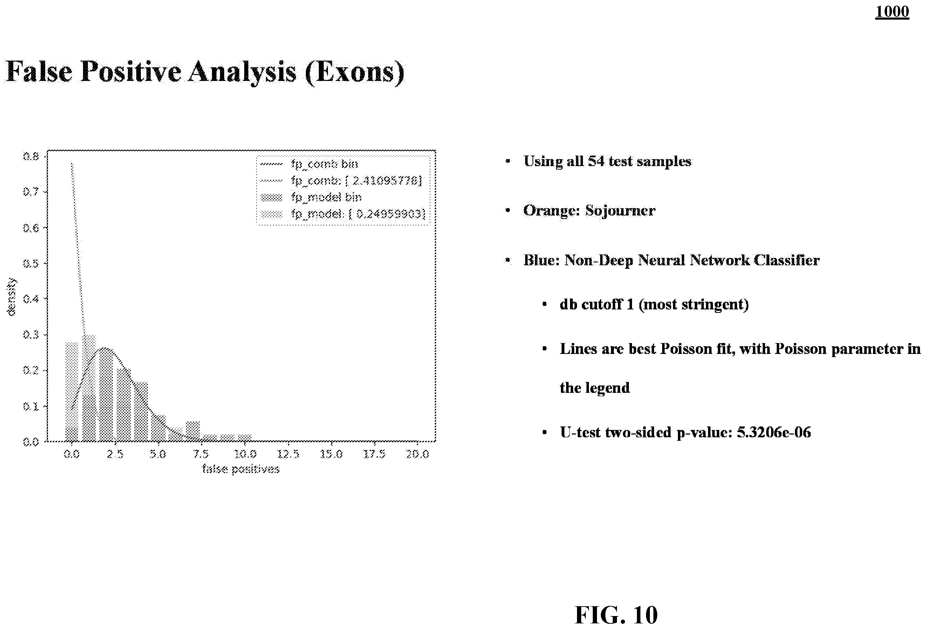

**FIG. 10** shows the improvement in false positive rate **1000** using Sojourner versus the non-deep neural network classifier when classifying variants over exons.

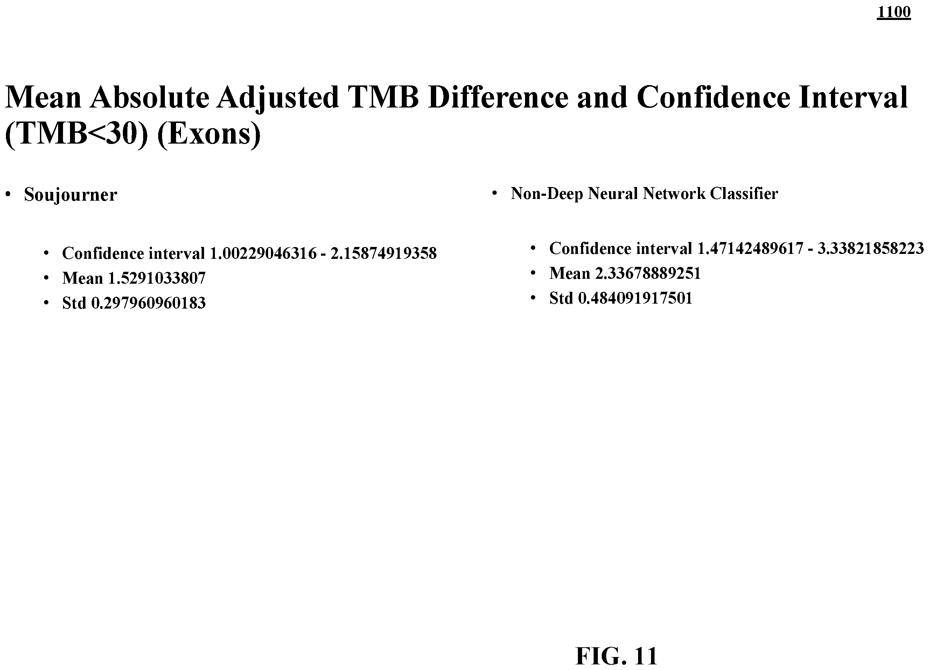

**FIG. 11** shows the mean absolute tumor mutational burden (TMB) error **1100** using Sojourner versus the non-deep neural network classifier when classifying variants over exons.

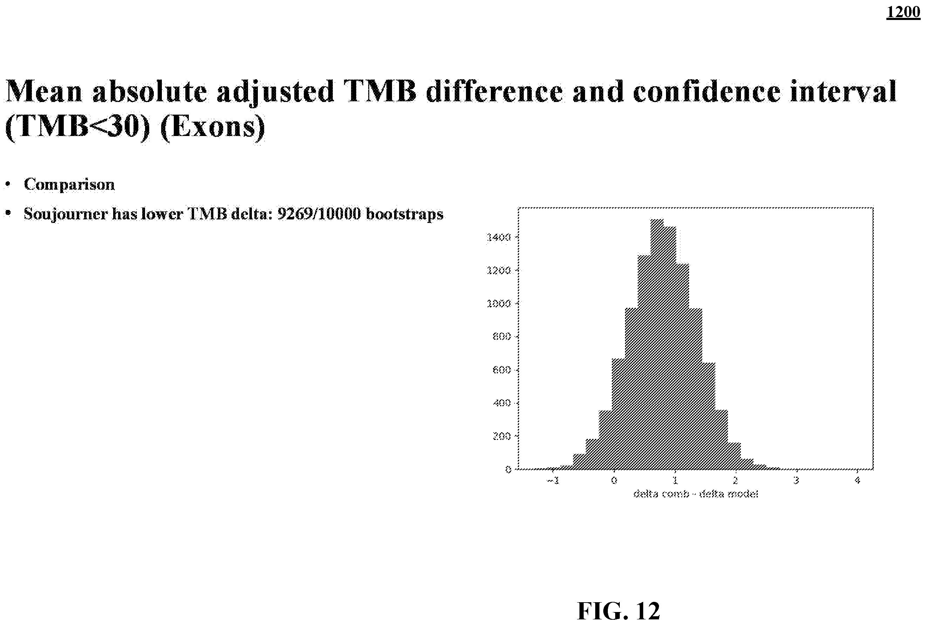

**FIG. 12** shows the improvement in mean absolute TMB error **1200** using Sojourner versus the non-deep neural network classifier when classifying variants over exons.

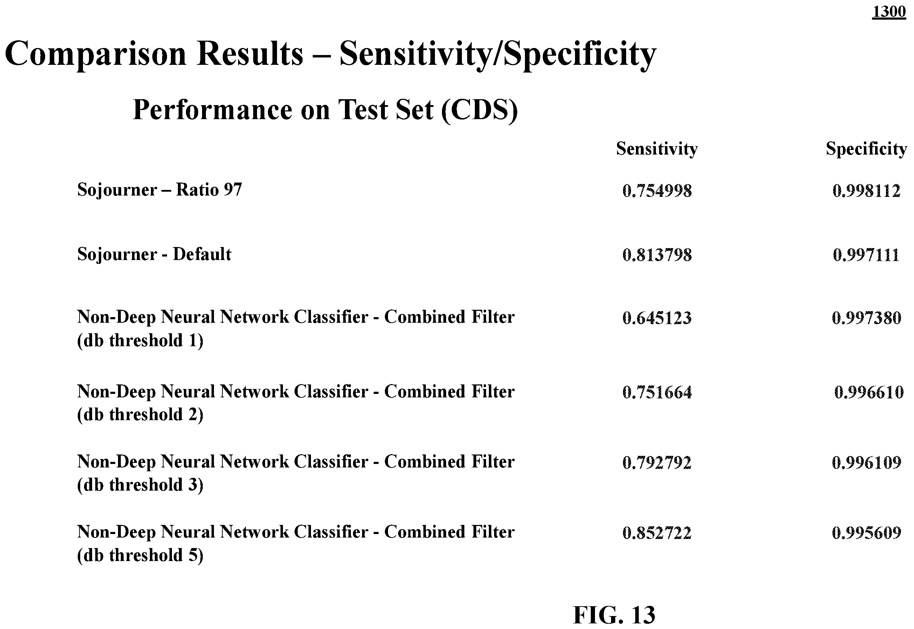

**FIG. 13** shows performance results **1300** of Sojourner on CDS (coding DNA sequence) data. These results, quantified by sensitivity and specificity, establish Sojourner's advantages and superiority over the non-deep neural network classifier.

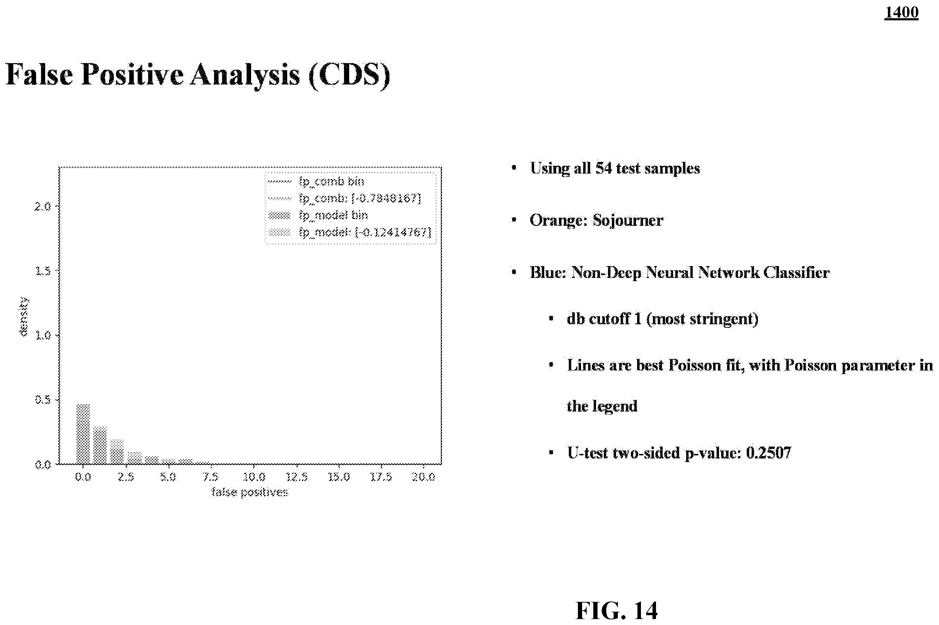

**FIG. 14** shows similar false positive rate **1400** using Sojourner versus the non-deep neural network classifier when classifying variants over coding regions.

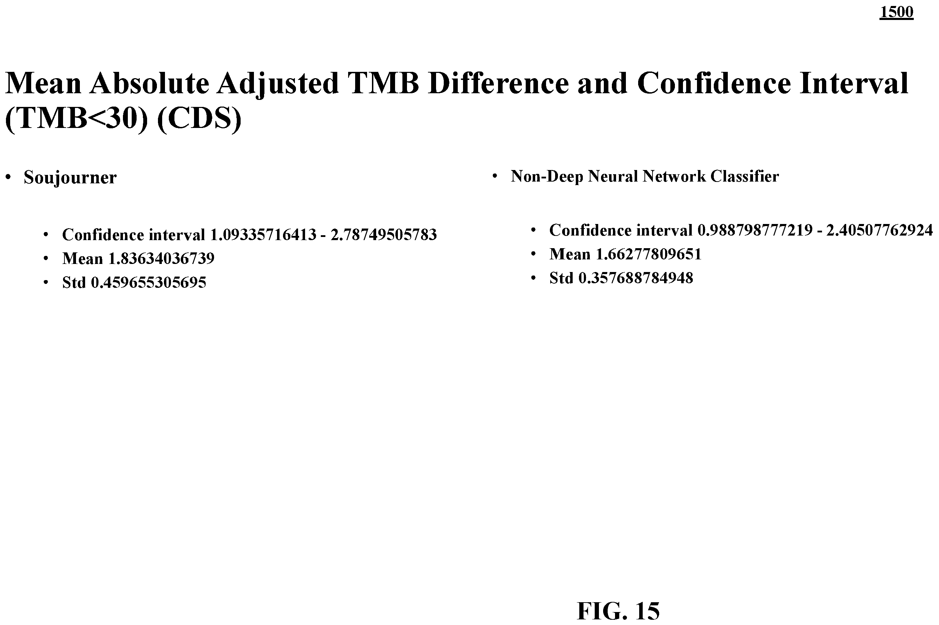

**FIG. 15** shows the mean absolute TMB error **1500** using Sojourner versus the non-deep neural network classifier when classifying variants over coding regions.

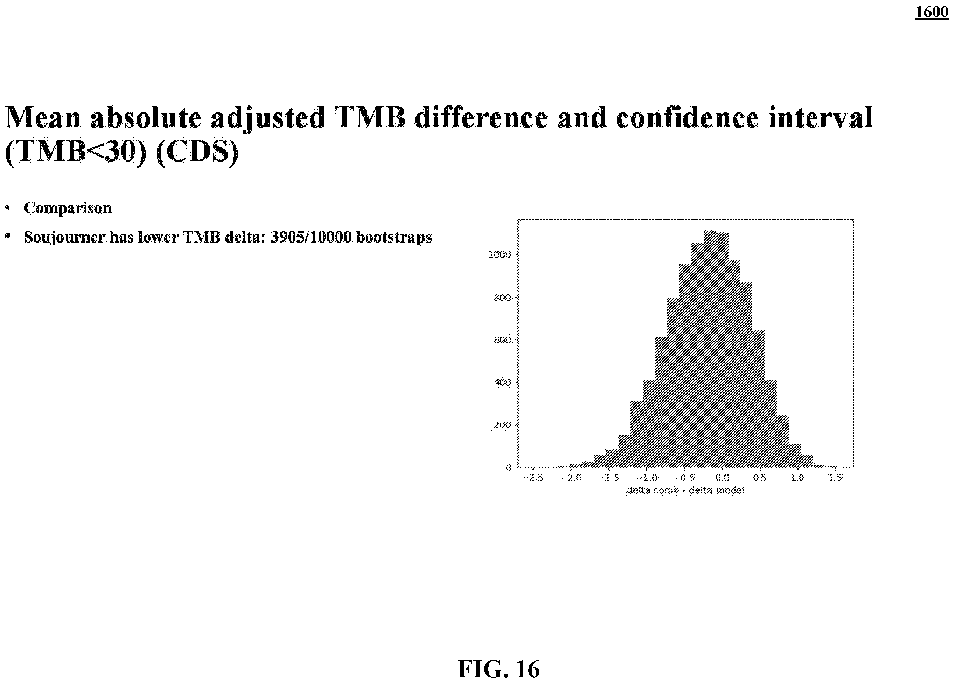

**FIG. 16** shows similar mean absolute TMB error **1600** using Sojourner versus the non-deep neural network classifier when classifying variants over exons.

### Computer System

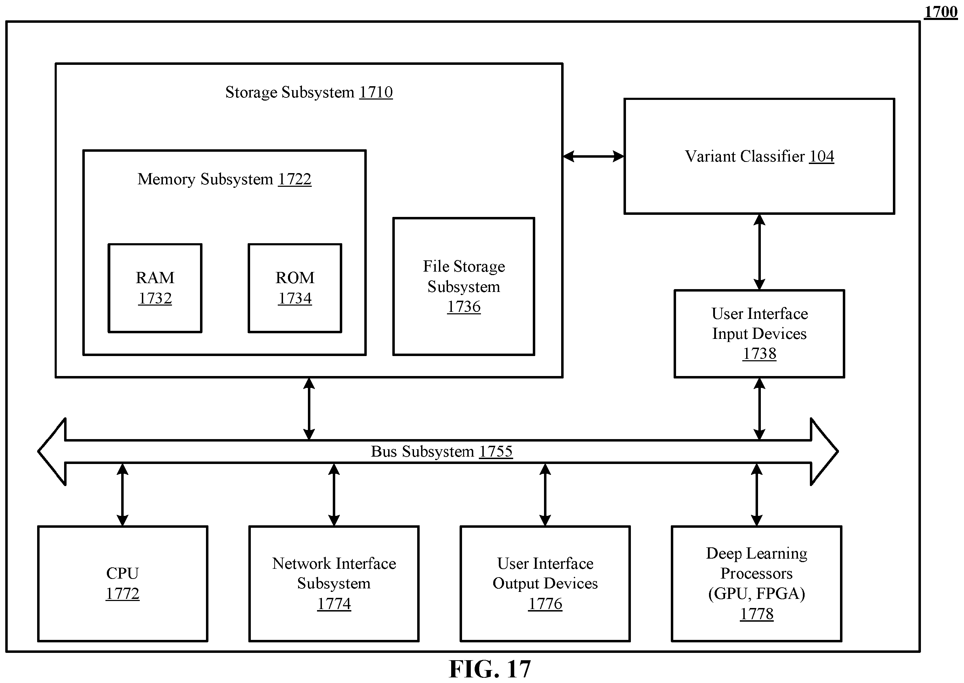

**FIG. 17** shows a computer system **1700** that can be used to implement the variant classifier **104.** Computer system **1700** includes at least one central processing unit (CPU) **1772** that communicates with a number of peripheral devices via bus subsystem **1755.** These peripheral devices can include a storage subsystem **1710** including, for example, memory devices and a file storage subsystem **1736,** user interface input devices **17317,** user interface output devices **1776,** and a network interface subsystem **1774.** The input and output devices allow user interaction with computer system **1700**. Network interface subsystem **1774** provides an interface to outside networks, including an interface to corresponding interface devices in other computer systems.

In one implementation, the variant classifier **104** is communicably linked to the storage subsystem 1710 and the user interface input devices **1738.**

User interface input devices **1738** can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system **1700.**

User interface output devices **1776** can include a display subsystem, a printer, a fax machine, or non-visual displays such as audio output devices. The display subsystem can include an LED display, a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system **1700** to the user or to another machine or computer system.

Storage subsystem **1710** stores programming and data constructs that provide the functionality of some or all of the modules and methods described herein. These software modules are generally executed by deep learning processors **1778**.

Deep learning processors **1778** can be graphics processing units (GPUs) or field-progranunable gate arrays (FPGAs). Deep learning processors **1778** can be hosted by a deep learning cloud platform such as Google Cloud Platform^{™}, Xilinx^{™}, and Cirrascale^{™}. Examples of deep learning processors **1778** include Google's Tensor Processing Unit (TPU)^{™}, rackmount solutions like GX4 Rackmount Series^{™}, GX17 Rackmount Series^{™}, NVIDIA DGX-1^{™}, Microsoft' Stratix V FPGA^{™}, Graphcore's Intelligent Processor Unit (IPU)^{™}, Qualcomm's Zeroth Platform^{™} with Snapdragon processors^{™}, NVIDIA's Volta^{™}, NVIDIA's DRIVE PX^{™}, NVIDIA's JETSON TX1/TX2 MODULE^{™}, Intel's Nirvana^{™}, Movidius VPU^{™}, Fujitsu DPI^{™}, ARM's DynamicIQ^{™}, IBM TrueNorth^{™}, and others.

Memory subsystem **1722** used in the storage subsystem **1710** can include a number of memories including a main random access memory (RAM) **1732** for storage of instructions and data during program execution and a read only memory (ROM) **1734** in which fixed instructions are stored. A file storage subsystem **1736** can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem **1736** in the storage subsystem **1710,** or in other machines accessible by the processor.

Bus subsystem **1755** provides a mechanism for letting the various components and subsystems of computer system **1700** communicate with each other as intended. Although bus subsystem **1755** is shown schematically as a single bus, alternative implementations of the bus subsystem can use multiple busses.

Computer system **1700** itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system **1700** depicted in **FIG. 17** is intended only as a specific example for purposes of illustrating the preferred embodiments of the present invention. Many other configurations of computer system **1700** are possible having more or less components than the computer system depicted in **FIG. 17****.**

### Particular Implementations

We describe a system and various implementations of a variant classifier that uses trained deep neural networks to predict whether a given variant is somatic of germline. One or more features of an implementation can be combined with the base implementation. Implementations that are not mutually exclusive are taught to be combinable. One or more features of an implementation can be combined with other implementations. This disclosure periodically reminds the user of these options. Omission from some implementations of recitations that repeat these options should not be taken as limiting the combinations taught in the preceding sections.

In one implementation, the technology disclosed presents a neural network-implemented system. The system comprises a variant classifier which runs on one or more processors operating in parallel and coupled to memory.

The variant classifier has: (i) a convolutional neural network and (ii) a fully-connected neural network, The convolutional neural network has at least two convolution layers and each of the convolution layers has at least five convolution filters trained over one thousand to millions of gradient update iterations to: (a) process an input sequence with a variant at a target position flanked by at least ten bases on each side, and (b) produce an intermediate convolved feature. In some implementations, each of the convolution layers has at least six convolution filters.

A metadata correlator correlates the variant with a set of metadata features which represent: (i) mutation characteristics of the variant, (ii) read mapping statistics of the variant, and (iii) occurrence frequency of the variant.

The fully-connected neural network has at least two fully-connected layers trained over the one thousand to millions of gradient update iterations to: (a) process a feature sequence derived from a combination of the intermediate convolved feature and the metadata features, and (b) output classification scores for likelihood that the variant is a somatic variant, a germline variant, or noise.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

The metadata correlator can be further configured to correlate the variant with an amino acid impact feature that specifies whether the variant is a nonsynonymous variant that changes a codon so as to produce a new codon which codes for a different amino acid.

The metadata correlator can be further configured to correlate the variant with a variant type feature that specifies type whether the variant is a single-nucleotide polymorphism, an insertion, or a deletion.

The metadata correlator can be further configured to correlate the variant with a read mapping statistic feature that specifies quality parameters of read mapping that identified the variant.

The metadata correlator can be further configured to correlate the variant with a population frequency feature that specifies allele frequencies of the variant in sequenced populations.

The metadata correlator can be further configured to correlate the variant with a sub-population frequency feature that specifies allele frequencies of the variant in ethnic sub-populations stratified from sequenced populations.

The metadata correlator can be further configured to correlate the variant with an evolutionary conservation feature that specifies conservativeness of the target position across multiple species.

The metadata correlator can be further configured to correlate the variant with a clinical significance feature that specifies the variant's clinical effect, drug sensitivity, and histocompatibility as determined from clinical tests.

The metadata correlator can be further configured to correlate the variant with a functional impact feature that specifies the variant's impact on functionality of a protein resulting from an amino acid substitution caused by the variant.

The metadata correlator can be further configured to correlate the variant with an ethnicity prediction feature that specifies likelihoods identifying ethnic makeup of an individual that provided a tumor sample associated with the variant.

The metadata correlator can be further configured to correlate the variant with a tumor frequency feature that specifies frequency of the variant in sequenced cancerous tumors.

The metadata correlator can be further configured to correlate the variant with an alternative allele feature that specifies at least one base mutated by the variant at the target position in a reference sequence.

The convolutional neural network and the fully-connected neural network of the variant classifier can be trained together end-to-end on five hundred thousand training examples from a first dataset of cancer-causing mutations, followed by training on fifty thousand training examples from a second dataset of cancer-causing mutations.

The convolutional neural network and the fully-connected neural network of the variant classifier can be tested together end-to-end on validation data held-out only from the second dataset.

Each of the convolution layers and the fully-connected layers can be followed by at least one rectified linear unit layer. Each of the convolution layers and the fully-connected layers can be followed by at least one batch normalization layer.

The variant can be flanked by at least 19 bases on each side. In another implementation, the variant can be flanked by at least 20 bases on each side.

The system can be further configured to comprise a concatenator that derives the feature sequence by concatenating the intermediate feature with the metadata features.

The metadata features can be encoded in a one-dimensional array. The input sequence can be encoded in an n-dimensional array, where n≥2.

Other implementations may include a non-transitory computer readable storage medium storing instructions executable by a processor to perform actions of the system described above. Each of the features discussed in the particular implementation section for other implementations apply equally to this implementation. As indicated above, all the other features are not repeated here and should be considered repeated by reference.

In another implementation, the technology disclosed presents a neural network-implemented method of variant classification.

The method includes processing an input sequence through a convolutional neural network to produce an intermediate convolved feature. The convolutional neural network has at least two convolution layers and each of the convolution layers has at least five convolution filters trained over one thousand to millions of gradient update iterations. In some implementations, each of the convolution layers has at least six convolution filters.

The input sequence has a variant at a target position flanked by at least ten bases on each side.

The method includes correlating the variant with a set of metadata features which represent: (i) mutation characteristics of the variant, (ii) read mapping statistics of the variant, and (iii) occurrence frequency of the variant.

The method includes processing a feature sequence through a fully-connected neural network to output classification scores for likelihood that the variant is a somatic variant, a germline variant, or noise. The fully-connected neural network has at least two fully-connected layers trained over the one thousand to millions of gradient update iterations. The feature sequence is derived from a combination of the intermediate convolved feature and the metadata features.

Other implementations may include a non-transitory computer readable storage medium (CRM) storing instructions executable by a processor to perform the method described above. Yet another implementation may include a system including memory and one or more processors operable to execute instructions, stored in the memory, to perform the method described above. Each of the features discussed in the particular implementation section for other implementations apply equally to this implementation. As indicated above, all the other features are not repeated here and should be considered repeated by reference.

In yet another implementation, the technology disclosed presents a neural network-implemented system. The system comprises a variant classifier which runs on one or more processors operating in parallel and coupled to memory.

The variant classifier has: (i) a convolutional neural network and (ii) a fully-connected neural network. The convolutional neural network is trained to process an input sequence and produce an intermediate convolved feature. The convolutional neural network has at least two convolution layers and each of the convolution layers has at least five convolution filters trained over one thousand to millions of gradient update iterations. In some implementations, each of the convolution layers has at least six convolution filters.

The input sequence has a variant at a target position flanked by at least ten bases on each side and has a set of metadata features correlated with the variant.

The metadata features represent: (i) mutation characteristics of the variant, (ii) read mapping statistics of the variant, and (iii) occurrence frequency of the variant.

The fully-connected neural network is trained to process the intermediate convolved feature and output classification scores for likelihood that the variant is a somatic variant a germline variant, or noise. The fully-connected neural network has at least two fully-connected layers trained over the one thousand to millions of gradient update iterations.

The system can be further configured to comprise a metadata correlator that correlates the variant with the metadata features.

Other implementations may include a non-transitory computer readable storage medium storing instructions executable by a processor to perform actions of the system described above. Each of the features discussed in the particular implementation section for other implementations apply equally to this implementation. As indicated above, all the other features are not repeated here and should be considered repeated by reference.

In yet further implementation, the technology disclosed presents a neural network-implemented method of variant classification.

The method includes processing an input sequence through a convolutional neural network to produce an intermediate convolved feature. The convolutional neural network has at least two convolution layers and each of the convolution layers has at least five convolution filters trained over one thousand to millions of gradient update iterations.

The input sequence has a variant at a target position flanked by at least ten bases on each side and has a set of metadata features correlated with the variant.

The metadata features represent: (i) mutation characteristics of the variant, (ii) read mapping statistics of the variant, and (iii) occurrence frequency of the variant.

The method includes processing the intermediate convolved feature through a fully-connected neural network to output classification scores for likelihood that the variant is a somatic variant, a germline variant, or noise. The fully-connected neural network has at least two fully-connected layers trained over the one thousand to millions of gradient update iterations.

Other implementations may include a non-transitory computer readable storage medium (CRM) storing instructions executable by a processor to perform the method described above. Yet another implementation may include a system including memory and one or more processors operable to execute instructions, stored in the memory, to perform the method described above. Each of the features discussed in the particular implementation section for other implementations apply equally to this implementation. ,

## Claims

1. A neural network-implemented system for classifying variants as somatic variants, germline variants, or noise comprising:
a variant classifier, running on one or more processors operating in parallel and coupled to memory, that has
a convolutional neural network having at least two convolution layers and each of the convolution layers having at least five convolution filters trained over one thousand to millions of gradient update iterations to
process an input sequence with a variant at a target position flanked by at least ten bases on each side, and
produce an intermediate convolved feature;
a metadata correlator that correlates the variant with a set of metadata features which represent
mutation characteristics of the variant,
read mapping statistics of the variant, and
occurrence frequency of the variant; and
a fully-connected neural network having at least two fully-connected layers trained over the one thousand to millions of gradient update iterations to
process a feature sequence derived by concatenating of the intermediate convolved feature and the set of metadata features, and
output, based on processing the feature sequence, a plurality of classification scores that each indicate a likelihood that the variant is a somatic variant, a germline variant, or noise.

2. The neural network-implemented system of claim 1, wherein the metadata correlator is further configured to correlate the variant with an amino acid impact feature that specifies whether the variant is a nonsynonymous variant that changes a codon so as to produce a new codon which codes for a different amino acid.

3. The neural network-implemented system of any of claims 1-2, wherein the metadata correlator is further configured to correlate the variant with a variant type feature that specifies type whether the variant is a single-nucleotide polymorphism, an insertion, or a deletion.

4. The neural network-implemented system of any of claims 1-3, wherein the metadata correlator is further configured to correlate the variant with a read mapping statistic feature that specifies quality parameters of read mapping that identified the variant.

5. The neural network-implemented system of any of claims 1-4, wherein the metadata correlator is further configured to correlate the variant with a population frequency feature that specifies allele frequencies of the variant in sequenced populations.

6. The neural network-implemented system of any of claims 1-5, wherein the metadata correlator is further configured to correlate the variant with a sub-population frequency feature that specifies allele frequencies of the variant in ethnic sub-populations stratified from sequenced populations.

7. The neural network-implemented system of any of claims 1-6, wherein the metadata correlator is further configured to correlate the variant with an evolutionary conservation feature that specifies conservativeness of the target position across multiple species.

8. The neural network-implemented system of any of claims 1-7, wherein the metadata correlator is further configured to correlate the variant with a clinical significance feature that specifies the variant's clinical effect, drug sensitivity, and histocompatibility as determined from clinical tests.

9. The neural network-implemented system of any of claims 1-8, wherein the metadata correlator is further configured to correlate the variant with a functional impact feature that specifies the variant's impact on functionality of a protein resulting from an amino acid substitution caused by the variant.

10. The neural network-implemented system of any of claims 1-9, wherein the metadata correlator is further configured to correlate the variant with an ethnicity prediction feature that specifies likelihoods identifying ethnic makeup of an individual that provided a tumor sample associated with the variant.

11. The neural network-implemented system of any of claims 1-10, wherein the metadata correlator is further configured to correlate the variant with a tumor frequency feature that specifies frequency of the variant in sequenced cancerous tumors.

12. The neural network-implemented system of any of claims 1-11, wherein the metadata correlator is further configured to correlate the variant with an alternative allele feature that specifies at least one base mutated by the variant at the target position in a reference sequence.

13. The neural network-implemented system of any of claims 1-12, wherein the convolutional neural network and the fully-connected neural network of the variant classifier are trained together end-to-end on five hundred thousand training examples from a first dataset of cancer-causing mutations, followed by training on fifty thousand training examples from a second dataset of cancer-causing mutations.

14. The neural network-implemented system of any of claims 1-13, wherein the fully-connected neural network includes a classification layer that outputs the plurality of classification scores by outputting a first probability of the variant being a somatic variant, a second probability of the variant being a germline variant, and a third probability of the variant being noise.

15. The neural network-implemented system of any of claim 14, wherein the third probability of the variant being noise comprises a probability of the variant being a mistaken variant call resulting from one or more errors in a sequencing process or a variant call application.

16. A neural network-implemented method implemented on a computer for classifying variants as somatic variants, germline variants, or noise including:
processing an input sequence through a convolutional neural network to produce an intermediate convolved feature, wherein
the convolutional neural network has at least two convolution layers and each of the convolution layers has at least five convolution filters trained over one thousand to millions of gradient update iterations, and
the input sequence has a variant at a target position flanked by at least ten bases on each side; correlating the variant with a set of metadata features which specify
mutation characteristics of the variant,
read mapping statistics of the variant, and
occurrence frequency of the variant; and
processing a feature sequence through a fully-connected neural network to output a plurality of classification scores that each indicate a likelihood that the variant is a somatic variant, a germline variant, or noise, wherein
the fully-connected neural network has at least two fully-connected layers trained over the one thousand to millions of gradient update iterations, and
the feature sequence is derived by concatenating the intermediate convolved feature and the set of metadata features.

17. A non-transitory computer readable storage medium impressed with computer program instructions for classifying variants as somatic variants, germline variants, or noise, the instructions, when executed on a processor, implement a method according to claim 16.

## Patentansprüche

1. Durch ein neuronales Netzwerk implementiertes System zum Klassifizieren von Varianten als somatische Varianten, Keimbahnvarianten oder Rauschen, umfassend:
ein Variantenklassifizierer, der auf einem oder mehreren parallel laufenden Prozessoren läuft und mit dem Speicher verbunden ist, umfassend
ein Convolutional Neural Network mit mindestens zwei Faltungsschichten, wobei jede der Faltungsschichten mindestens fünf Faltungsfilter aufweist, die über tausend bis Millionen von Gradientenaktualisierungsiterationen trainiert wurden, um
eine Eingabesequenz mit einer Variante an einer Zielposition zu verarbeiten, die auf jeder Seite von mindestens zehn Basen flankiert wird, und
ein gefaltetetes Zwischenmerkmal zu erzeugen;
Metadatenkorrelator, der die Variante mit einem Satz von Metadatenmerkmalen korreliert, die
Mutationsmerkmale der Variante,
Lesezuordnungsstatistiken der Variante und
Auftretenshäufigkeit der Variante darstellen; und
ein vollständig verbundenes neuronales Netzwerk mit mindestens zwei vollständig verbundenen Schichten, das über tausend bis Millionen von Gradientenaktualisierungsiterationen trainiert wurde, um
eine Merkmalssequenz, die durch Verketten des gefalteten Zwischenmerkmals und des Satzes von Metadatenmerkmalen abgeleitet wird, zu verarbeiten und
basierend auf der Verarbeitung der Merkmalssequenz eine Vielzahl von Klassifizierungswerten auszugeben, die jeweils eine Wahrscheinlichkeit angeben, dass die Variante eine somatische Variante, eine Keimbahnvariante oder Rauschen ist.

2. Durch ein neuronales Netzwerk implementiertes System nach Anspruch 1, wobei der Metadatenkorrelator ferner dazu konfiguriert ist, die Variante mit einem Aminosäure-Auswirkungsmerkmal zu korrelieren, das angibt, ob die Variante eine nicht-synonyme Variante ist, die ein Codon ändert, um ein neues Codon zu erzeugen, das für eine andere Aminosäure kodiert.

3. Durch ein neuronales Netzwerk implementiertes System nach einem der Ansprüche 1 bis 2, wobei der Metadatenkorrelator ferner dazu konfiguriert ist, die Variante mit einem Variantentypmerkmal zu korrelieren, das den Typ angibt, ob die Variante ein Einzelnukleotidpolymorphismus, eine Insertion oder eine Deletion ist.

4. Durch ein neuronales Netzwerk implementiertes System nach einem der Ansprüche 1 bis 3, wobei der Metadatenkorrelator ferner dazu konfiguriert ist, die Variante mit einem Lesezuordnungsstatistikmerkmal zu korrelieren, das Qualitätsparameter der Lesezuordnung angibt, die die Variante identifiziert hat.

5. Durch ein neuronales Netzwerk implementiertes System nach einem der Ansprüche 1 bis 4, wobei der Metadatenkorrelator ferner dazu konfiguriert ist, die Variante mit einem Populationshäufigkeitsmerkmal zu korrelieren, das Allelhäufigkeiten der Variante in sequenzierten Populationen angibt.

6. Durch ein neuronales Netzwerk implementiertes System nach einem der Ansprüche 1 bis 5, wobei der Metadatenkorrelator ferner dazu konfiguriert ist, die Variante mit einem Subpopulationshäufigkeitsmerkmal zu korrelieren, das Allelhäufigkeiten der Variante in ethnischen Subpopulationen angibt, die aus sequenzierten Populationen stratifiziert wurden.

7. Durch ein neuronales Netzwerk implementiertes System nach einem der Ansprüche 1 bis 6, wobei der Metadatenkorrelator ferner dazu konfiguriert ist, die Variante mit einem evolutionären Erhaltungsmerkmal zu korrelieren, das die Konservativität der Zielposition über mehrere Arten hinweg angibt.

8. Durch ein neuronales Netzwerk implementiertes System nach einem der Ansprüche 1 bis 7, wobei der Metadatenkorrelator ferner dazu konfiguriert ist, die Variante mit einem Merkmal klinischer Signifikanz zu korrelieren, das die klinische Wirkung, Arzneimittelempfindlichkeit und Histokompatibilität der Variante angibt, wie sie in klinischen Tests ermittelt wurden.

9. Durch ein neuronales Netzwerk implementiertes System nach einem der Ansprüche 1 bis 8, wobei der Metadatenkorrelator ferner dazu konfiguriert ist, die Variante mit einem funktionellen Auswirkungsmerkmal zu korrelieren, das die Auswirkung der Variante auf die Funktionalität eines Proteins angibt, die sich aus einem durch die Variante verursachten Aminosäureaustausch ergibt.

10. Durch ein neuronales Netzwerk implementiertes System nach einem der Ansprüche 1 bis 9, wobei der Metadatenkorrelator ferner dazu konfiguriert ist, die Variante mit einem Merkmal zur Vorhersage der ethnischen Herkunft zu korrelieren, die Wahrscheinlichkeiten zur Identifizierung der ethnischen Zusammensetzung einer Person angibt, die eine mit der Variante verbundene Tumorprobe bereitgestellt hat.

11. Durch ein neuronales Netzwerk implementiertes System nach einem der Ansprüche 1 bis 10, wobei der Metadatenkorrelator ferner dazu konfiguriert ist, die Variante mit einem Tumorhäufigkeitsmerkmal zu korrelieren, das die Häufigkeit der Variante in sequenzierten Krebstumoren angibt.

12. Durch ein neuronales Netzwerk implementiertes System nach einem der Ansprüche 1 bis 11, wobei der Metadatenkorrelator ferner dazu konfiguriert ist, die Variante mit einem alternativen Allelmerkmal zu korrelieren, das mindestens eine durch die Variante an der Zielposition in einer Referenzsequenz mutierte Base angibt.

13. Durch ein neuronales Netzwerk implementiertes System nach einem der Ansprüche 1 bis 12, wobei das Convolutional Neural Network und das vollständig verbundene neuronale Netzwerk des Variantenklassifizierers gemeinsam Endezu-Ende anhand von fünfhunderttausend Trainingsbeispielen aus einem ersten Datensatz krebserregender Mutationen trainiert werden, gefolgt von einem Training anhand von fünfzigtausend Trainingsbeispielen aus einem zweiten Datensatz krebserregender Mutationen.

14. Durch ein neuronales Netzwerk implementiertes System nach einem der Ansprüche 1 bis 13, wobei das vollständig verbundene neuronale Netzwerk eine Klassifizierungsschicht beinhaltet, die die Vielzahl von Klassifizierungswerten ausgibt, indem sie eine erste Wahrscheinlichkeit, dass die Variante eine somatische Variante ist, eine zweite Wahrscheinlichkeit, dass die Variante eine Keimbahnvariante ist, und eine dritte Wahrscheinlichkeit, dass die Variante Rauschen ist, ausgibt.

15. Durch ein neuronales Netzwerk implementiertes System nach Anspruch 14, wobei die dritte Wahrscheinlichkeit, dass die Variante Rauschen ist, eine Wahrscheinlichkeit umfasst, dass die Variante ein irrtümlicher Variantenaufruf ist, der sich aus einem oder mehreren Fehlern in einem Sequenzierungsprozess oder einer Variantenaufrufanwendung ergibt.

16. Durch ein neuronales Netzwerk und auf einem Computer implementiertes Verfahren zum Klassifizieren von Varianten als somatische Varianten, Keimbahnvarianten oder
Rauschen, einschließlich:
Verarbeiten einer Eingabesequenz durch ein Convolutional Neural Network, um ein gefaltetetes Zwischenmerkmal zu erzeugen, wobei
das Convolutional Neural Network mindestens zwei Faltungsschichten aufweist und jede der Faltungsschichten mindestens fünf Faltungsfilter aufweist, die über tausend bis Millionen von Gradientenaktualisierungsiterationen trainiert wurden, und
die Eingabesequenz an einer Zielposition eine Variante aufweist, die auf jeder Seite von mindestens zehn Basen flankiert wird; Korrelieren der Variante mit einem Satz von Metadatenmerkmalen, die
Mutationsmerkmale der Variante,
Lesezuordnungsstatistiken der Variante und
Auftretenshäufigkeit der Variante darstellen; und
Verarbeiten einer Merkmalssequenz durch ein vollständig verbundenes neuronales Netzwerk, um eine Vielzahl von Klassifizierungswerten auszugeben, die jeweils eine Wahrscheinlichkeit angeben, dass die Variante eine somatische Variante, eine Keimbahnvariante oder Rauschen ist, wobei
das vollständig verbundene neuronale Netzwerk mindestens zwei vollständig verbundene Schichten aufweist, das über tausend bis Millionen von Gradientenaktualisierungsiterationen trainiert wurde, und
die Merkmalssequenz durch Verketten des gefalteten Zwischenmerkmals und des Satzes von Metadatenmerkmalen abgeleitet wird.

17. Nicht flüchtiges, computerlesbares Speichermedium, das mit Computerprogrammanweisungen zum Klassifizieren von Varianten als somatische Varianten, Keimbahnvarianten oder Rauschen versehen ist, wobei die Anweisungen, wenn sie auf einem Prozessor ausgeführt werden, ein Verfahren nach Anspruch 16 implementieren.

## Revendications

1. Système mis en œuvre par un réseau neuronal pour classer des variants en variants somatiques, variants germinaux, ou bruit comprenant :
un classificateur de variants, en exécution sur un ou plusieurs processeurs fonctionnant en parallèle et couplé à une mémoire, qui a
un réseau neuronal convolutif ayant au moins deux couches de convolution et chacune des couches de convolution comportant au moins cinq filtres de convolution entraînés sur un millier à des millions d'itérations de mise à jour de gradient pour
traiter une séquence d'entrée avec un variant à une position cible flanquée d'au moins dix bases de chaque côté, et
produire une caractéristique convoluée intermédiaire ;
un corrélateur de métadonnées qui établit une corrélation entre le variant et un ensemble de caractéristiques de métadonnées représentant
des caractéristiques de mutation du variant,
des statistiques de cartographie de lecture du variant, et
une fréquence d'apparition du variant ; et
un réseau neuronal entièrement connecté ayant au moins deux couches entièrement connectées, entraîné sur les mille à millions d'itérations de mise à jour de gradient pour
traiter une séquence de caractéristiques dérivée de la concaténation de la caractéristique convoluée intermédiaire et de l'ensemble de caractéristiques de métadonnées, et
générer, sur la base du traitement de la séquence de caractéristiques, une pluralité de scores de classification qui indiquent chacun une probabilité que le variant soit un variant somatique, un variant germinal, ou du bruit.

2. Système mis en œuvre par un réseau neuronal selon la revendication 1, dans lequel le corrélateur de métadonnées est en outre configuré pour corréler le variant avec une caractéristique d'impact sur l'acide aminé qui spécifie si le variant est un variant non synonyme qui modifie un codon de manière à produire un nouveau codon qui code pour un acide aminé différent.

3. Système mis en œuvre par un réseau neuronal selon l'une quelconque des revendications 1 à 2, dans lequel le corrélateur de métadonnées est en outre configuré pour corréler le variant avec une caractéristique de type de variant qui spécifie si le variant est un polymorphisme d'un seul nucléotide, une insertion, ou une délétion.

4. Système mis en œuvre par un réseau neuronal selon l'une quelconque des revendications 1 à 3, dans lequel le corrélateur de métadonnées est en outre configuré pour corréler le variant avec une caractéristique statistique de cartographie de lecture qui spécifie des paramètres de qualité de cartographie de lecture qui a identifié le variant.

5. Système mis en œuvre par un réseau neuronal selon l'une quelconque des revendications 1 à 4, dans lequel le corrélateur de métadonnées est en outre configuré pour corréler le variant avec une caractéristique de fréquence de population qui spécifie des fréquences d'allèles du variant dans des populations séquencées.

6. Système mis en œuvre par un réseau neuronal selon l'une quelconque des revendications 1 à 5, dans lequel le corrélateur de métadonnées est en outre configuré pour corréler le variant avec une caractéristique de fréquence de sous-population qui spécifie des fréquences d'allèles du variant dans des sous-populations ethniques stratifiées à partir de populations séquencées.

7. Système mis en œuvre par un réseau neuronal selon l'une quelconque des revendications 1 à 6, dans lequel le corrélateur de métadonnées est en outre configuré pour corréler le variant avec une caractéristique de conservation évolutive qui spécifie la conservativité de la position cible dans de multiples espèces.

8. Système mis en œuvre par un réseau neuronal selon l'une quelconque des revendications 1 à 7, dans lequel le corrélateur de métadonnées est en outre configuré pour corréler le variant avec une caractéristique de signification clinique qui spécifie l'effet clinique, la sensibilité aux médicaments, et l'histocompatibilité du variant, tels que déterminés à partir d'essais cliniques.

9. Système mis en œuvre par un réseau neuronal selon l'une quelconque des revendications 1 à 8, dans lequel le corrélateur de métadonnées est en outre configuré pour corréler le variant avec une caractéristique d'impact fonctionnel qui spécifie l'impact du variant sur la fonctionnalité d'une protéine résultant d'une substitution d'acide aminé provoquée par le variant.

10. Système mis en œuvre par un réseau neuronal selon l'une quelconque des revendications 1 à 9, dans lequel le corrélateur de métadonnées est en outre configuré pour corréler le variant avec une caractéristique de prédiction d'ethnicité qui spécifie des probabilités d'identification de la composition ethnique d'un individu qui a fourni un échantillon de tumeur associé au variant.

11. Système mis en œuvre par un réseau neuronal selon l'une quelconque des revendications 1 à 10, dans lequel le corrélateur de métadonnées est en outre configuré pour corréler le variant avec une caractéristique de fréquence de tumeur qui spécifie une fréquence du variant dans des tumeurs cancéreuses séquencées.

12. Système mis en œuvre par un réseau neuronal selon l'une quelconque des revendications 1 à 11, dans lequel le corrélateur de métadonnées est en outre configuré pour corréler le variant avec une caractéristique d'allèles alternatifs qui spécifie au moins une base mutée par le variant à la position cible dans une séquence de référence.

13. Système mis en œuvre par un réseau neuronal selon l'une quelconque des revendications 1 à 12, dans lequel le réseau neuronal convolutif et le réseau neuronal entièrement connecté du classificateur de variants sont entraînés ensemble de bout en bout sur cinq cent mille exemples d'entraînement provenant d'un premier ensemble de données de mutations cancérigènes, suivi d'un entraînement sur cinquante mille exemples d'entraînement provenant d'un second ensemble de données de mutations cancérigènes.

14. Système mis en œuvre par un réseau neuronal selon l'une quelconque des revendications 1 à 13, dans lequel le réseau neuronal entièrement connecté comporte une couche de classification qui génère la pluralité de scores de classification en générant une première probabilité que le variant soit un variant somatique, une deuxième probabilité que le variant soit un variant germinal, et une troisième probabilité que le variant soit un bruit.

15. Système mis en œuvre par un réseau neuronal selon l'une quelconque de la revendication 14, dans lequel la troisième probabilité que le variant soit un bruit comprend une probabilité que le variant soit un appel de variant erroné résultant d'une ou de plusieurs erreurs dans un processus de séquençage ou une application d'appel de variant.

16. Procédé mis en œuvre par un réseau neuronal sur un ordinateur pour classer des variants en variants somatiques, variants germinaux, ou
un bruit comportant :
le traitement d'une séquence d'entrée à travers un réseau neuronal convolutif pour produire une caractéristique convolutive intermédiaire, dans lequel
le réseau neuronal convolutif a au moins deux couches de convolution et chacune des couches de convolution a au moins cinq filtres de convolution entraînés sur un millier à des millions d'itérations de mise à jour de gradient, et
la séquence d'entrée a un variant à une position cible flanquée d'au moins dix bases de chaque côté ; la corrélation du variant avec un ensemble de caractéristiques de métadonnées qui spécifient
des caractéristiques de mutation du variant,
des statistiques de cartographie de lecture du variant, et
une fréquence d'apparition du variant ; et
le traitement d'une séquence de caractéristiques par le biais d'un réseau neuronal entièrement connecté afin de générer une pluralité de scores de classification qui indiquent chacun une probabilité que le variant soit un variant somatique, un variant germinal, ou un bruit, dans lequel
le réseau neuronal entièrement connecté a au moins deux couches entièrement connectées, entraîné sur les mille à millions d'itérations de mise à jour de gradient, et
la séquence de caractéristiques est dérivée de la concaténation de la caractéristique convoluée intermédiaire et de l'ensemble de caractéristiques de métadonnées.

17. Support de stockage non transitoire lisible par ordinateur sur lequel sont imprimées des instructions de programme informatique pour classer des variants en variants somatiques, variants germinaux, ou bruit, les instructions, lorsqu'elles sont exécutées sur un processeur, mettent en œuvre un procédé selon la revendication 16.
